(19) Europäisches Patentamt
European Patent Office
Office européen des brevets

(11) EP 3 555 301 B1

(12) **EUROPEAN PATENT SPECIFICATION**

(45) Date of publication and mention of the grant of the patent:
**09.09.2020 Bulletin 2020/37**

(21) Application number: **17826094.9**

(22) Date of filing: **15.12.2017**

(51) Int Cl.:
*C12P 19/02* (2006.01) *C12N 9/24* (2006.01)

(86) International application number:
**PCT/US2017/066737**

(87) International publication number:
**WO 2018/112376 (21.06.2018 Gazette 2018/25)**

(54) **METHOD FOR INCREASING THE PRODUCTION OF ETHANOL FROM CORN FIBER IN A STARCH HYDROLYSIS PROCESS BY USING GH31 ALPHA-GLUCOSIDASES**

VERFAHREN ZUR ERHÖHUNG DER PRODUKTION VON ETHANOL AUS MAISFASERN IN EINEM VERFAHREN ZUR HYDROLYSE VON STÄRKE UNTER VERWENDUNG VON GH31 ALPHA-GLUCOSIDASEN

PROCÉDÉ POUR AUGMENTER LA PRODUCTION D'ÉTHANOL À PARTIR DE FIBRE DE MAÏS DANS UN PROCÉDÉ D'HYDROLYSE D'AMIDON A L'AIDE DE ALPHA-GLUCOSIDASES GH31

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.12.2016 US 201662434883 P**

(43) Date of publication of application:
**23.10.2019 Bulletin 2019/43**

(73) Proprietor: **Danisco US Inc.**
**Palo Alto, California 94304 (US)**

(72) Inventors:
- **MILLER, Jeffrey V.**
  **Palo Alto, California 94304 (US)**
- **KELEMEN, Bradley Roger**
  **Palo Alto, California 94304 (US)**
- **VAN DER KLEY, Pim**
  **Palo Alto, California 94304 (US)**
- **NIKOLAEV, Igor**
  **Palo Alto, California 94304 (US)**
- **CHOW, Su Yin Marina**
  **Palo Alto, California 94304 (US)**
- **TSE, Monica**
  **Palo Alto, California 94304 (US)**
- **GE, Jing**
  **Palo Alto, California 94304 (US)**
- **TANG, Zhongmei**
  **Palo Alto, California 94304 (US)**
- **CROTTY, Kirstin Yasuko Nosé**
  **Palo Alto, California 94304 (US)**

(74) Representative: **Mewburn Ellis LLP**
**Aurora Building**
**Counterslip**
**Bristol BS1 6BX (GB)**

(56) References cited:
**WO-A1-2016/138315 WO-A2-2014/202622**

- **DATABASE UniParc [Online] 2013, N.N.: "Byssochlamys spectabilis (strain No. 5 / NBRC 109023) (Paecilomyces variotii)", XP002779769, Database accession no. UPI0003CCB1B9**
- **OKUYAMA ET AL: "Alpha-glucosidases and alpha-1,4-glucan lyases: structures, functions, and physiological actions", CELLULAR AND MOLECULAR LIFE SCIENCES, vol. 73, 30 April 2016 (2016-04-30), pages 2727-2751, XP035988677, cited in the application**
- **BIELY ET AL: "Towards enzymatic breakdown of complex plant xylan structures: state of the art", BIOTECHNOLOGY ADVANCES, vol. 34, 9 September 2016 (2016-09-09), pages 1260-1274, XP029765719,**
- **JUNG ET AL: "Broad substrate specificity of a hyperthermophilic alpha-glucosidase from Pyrobaculum arsenaticum", FOOD SCIENCE AND BIOTECHNOLOGY, vol. 25, 31 December 2016 (2016-12-31), pages 1665-1669, XP002779770,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 555 301 B1

## Description

### TECHNICAL FIELD

The present methods relate to the use of a GH31 α-glucosidase to increase the production of ethanol from a corn fiber material derived from a predominantly corn kernel feedstock.

### BACKGROUND

Many commercial fermentation process are designed to maximize the conversion of plant-derived complex carbohydrates into simple fermentable sugars. These sugars may be simultaneously or sequentially utilized by microorganisms to produce commercially-valuable biochemicals or biofuels. One well-known such process is the conversion of starch into glucose followed by yeast fermentation to produce ethanol. A commonly used feedstock is corn kernels, which may include some com stover, resulting in a feedstock that is predominantly starch but contains a significant amount of corn fiber.

Com fiber is sometimes separated from corn carbohydrates by mechanical fractionation, resulting in essentially two (or more) different feedstocks having different chemical and enzymatic processing requirements. In addition, corn-ethanol processing generates by-products that include non-fermentable plant materials. There is considerable interest in treating corn fiber and corn processing by-products that contain corn fiber to extract more fermentable sugars, thereby improving the overall yield of commercially valuable biochemicals or biofuels.

WO 2016/138315 refers to alpha-glucosidases from Rasamsonia and uses thereof. UniParc UPI0003CCB1B9 discloses a Paecilomyces variotii sequence that is about 93% identical with SEQ ID NO:3 of the present Application.

### SUMMARY

The present invention refers to (i-ii) methods for increasing the production of fermentable sugars, comprising contacting a GH31 enzyme with pretreated corn fiber or stillage, and (iii) Paecilomyces variotii polypeptides and corresponding polynucleotides.

### BRIEF DESCRIPTION OF THE DRAWINGS

Figure 1 is a plasmid map of pTTT-PvaGlu1.

Figure 2 is a graph showing the amount of glucose (mg/mL) released from a whole stillage substrate using various amounts of different α-glucosidases (μg/g dry solids) at pH 5 and 50°C for 24 hours.

### DETAILED DESCRIPTION

#### I. Introduction

The present invention, as defined in the claims, relate to an improvement on current methods that utilize enzymes to release fermentable sugars from plant-derived carbohydrates. Specifically, the compositions and methods involve the addition a GH31 α-glucosidase, which has a complex substrate specificity, to corn fiber derived from a predominantly corn kernel feedstock that has been pretreated in preparation for enzymatic digestion. The present methods should not be confused with the addition of α-glucosidase to a cellulosic feedstock in a cellulose-based biofuel process.

#### II. Definitions

Prior to describing the present compositions and methods in detail, the following terms are defined for clarity. Terms not defined should be accorded their ordinary meanings as used in the relevant art.

As used herein, an "a-glucosidase" (EC 3.2.1.20) is an enzyme capable of the hydrolysis of terminal, non-reducing, α (1-4)-linked -D-glucose residues from a substrate, with the resulting release of D-glucose. α-glucosidases are not to be confused with β-glucosidases (E.C. 3.2.1.21) or other carbohydrate-processing enzymes, including but not limited to α-amylases (EC 3.2.1.1), β-amylases (EC 3.2.1.2), glucoamylases (EC 3.2.1.3), trehalases (EC 3.2.1.28), pullulanses (EC 3.2.1.41), and other enzymes having predominantly different substrate specificities.

In the context of the invention, the α-glucosidase is a GH31 enzyme.

As used herein, "background α-glucosidase activity," and similar terms, refer to enzyme activity that is not attributable to the addition of exogenous α-glucosidase to plant fiber.

As used herein, "physical treatment," or similar expressions, refer to mechanical and/or thermal treatments, which may or may not result in chemical modifications, that modifying a fiber substrate to increase its suitability for digestion by enzymes, particularly α-glucosidase.

As used herein, "thermal pretreatment" refers to exposure to a temperature suitable to evaporate alcohol, particularly ethanol, as in the case of recovering fuel alcohol from a fermentation process, or to otherwise modify a fermentation substrate by heat, so as to modify the substrate to increase enzymatic digestion. Thermal pretreatment typically involves a temperature of at least 80°C.

As used herein, "chemical treatment" refers to non-enzymatic modification of a substrate, such as by acid or alkaline treatment (*i.e.,* pH adjustment), exposure to oxidizing or reducing agents, and the like.

As used herein, a "predominantly corn kernel feedstock" is a feedstock containing at least 80%, at least 85%, at least 90%, at least 95%, at least 97% and even at least 98% corn kernels. The remaining material may include corn stover, other plant components, insects and the like.

As used herein, a "corn fiber product" is material produced as a result of processing a predominantly corn kernel-based substrate in a typical fuel ethanol processing plant, where the steps of starch hydrolysis and fermentation are performed to produce ethanol. An exemplary corn fiber product is whole stillage.

As used herein, "wet distillers' grains (WDG)" primarily contain unfermented grain residues, specifically protein, fiber, fat and up to 70% moisture.

As used herein, "dried distillers' grains with solubles (DDGS) is WDG that has been dried with the concentrated thin stillage (liquid remaining after boiling of the ethanol) to about 10-12 percent moisture. The fiber content can be up to about 5-50%.

As used herein, the singular articles "a," "an," and "the" encompass the plural referents unless the context clearly dictates otherwise. The following abbreviations/acronyms have the following meanings unless otherwise specified:

| | |
|---|---|
| EC | enzyme commission |
| CAZy | carbohydrate active enzyme |
| w/v | weight/volume |
| w/w | weight/weight |
| v/v | volume/volume |
| wt% | weight percent |
| °C | degrees Centigrade |
| g or gm | gram |
| μg | microgram |
| mg | milligram |
| kg | kilogram |
| μL and μl | microliter |
| mL and ml | milliliter |
| mm | millimeter |
| μm | micrometer |
| mol | mole |
| mmol | millimole |
| M | molar |
| mM | millimolar |
| μM | micromolar |
| nm | nanometer |
| U | unit |
| ppm | parts per million |
| hr and h | hour |
| EtOH | ethanol |

### III. α-glucosidases

All embodiments of the present methods relate to the use of a GH31 α-glucosidase for improving ethanol production from corn fiber derived from a predominantly corn kernel feedstock (at least 80% corn kernels).

α-glucosidases (EC 3.2.1.20) hydrolyze terminal, non-reducing α-1,4- linked glucose residues in various substrates, releasing glucose. They degrade disaccharides and oligosaccharides quickly while polysaccharides are attacked more slowly, α-glucosidases display broad substrate specificity for α-glucosides, which include panose, isomaltose, isopanose, maltotriose, turanose, and maltose. Aside from being able to hydrolyze synthetic α-glucoside and oligosaccharide substrates, α-glucosidases also can hydrolyze α-glucans such as soluble starch and glycogen.

α-glucosidases can be divided into three types based on substrate specificity. Type 1 α-glucosidases hydrolyze heterogeneous substrates, such as aryl glucosides and sucrose, more efficiently than they hydrolyze maltose. Type II α-glucosidases prefer maltose and isomaltose as substrates, and have low activities toward aryl glucosides. Type III α-glucosdiases have the same substrate specificity as type II but can additionally hydrolyze polysaccharides such as amylose and starch.

α-glucosidase activity can be found in a wide variety of organisms. α-glucosidases from bacteria, yeast, and insects typically belong to the GH13 class, and have four conserved sequence regions, α-glucosidases from plants, animals, molds, and two species of bacteria are members of GH31 class. Additional α-glucosidase of members of GH97 class.

In the present methods, the α-glucosidase are of the GH31 class, as described in, *e.g.,* Okuyama, M. et al. (2016) Cell. Mol. Life Sci. 73:2727-51. Exemplary GH31 enzymes are E1, E2, E3 and E4, having the amino acid sequences of SEQ ID NO: 1, SEQ ID NO: 2, SEQ ID NO: 3 and SEQ ID NO: 4, respectively, as shown, below:

E1 GH31 α-glucosidase (SEQ ID NO: 1):

STTAPSQPQFTIPASADVGAQLIANIDDPQAADAQSVCPGYKASKVQHNSRGFTASLQLAGRP
CNVYGTDVESLTLSVEYQDSDRLNIQILPTHVDSTNASWYFLSENLVPRPKASLNASVSQSDL
FVSWSNEPSFNFKVIRKATGDALFSTEGTVLVYENQFIEFVTALPEEYNLYGLGEHITQFRLQ
RNANLTIYPSDDGTPIDQNLYGQHPFYLDTRYYKGDRQNGSYIPVKSSEADASQDYISLSHGV
FLRNSHGLEILLRSQKLIWRTLGGGIDLTFYSGPAPADVTRQYLTSTVGLPAMQQYNTLGFHQ

CRWGYNNWSDLADVVANFEKFEIPLEYIWTDIDYMHGYRNFDNDQHRFSYSEGDEFLSKLHES
GRYYVPIVDAALYIPNPENASDAYATYDRGAADDVFLKNPDGSLYIGAVWPGYTVFPDWHHPK
AVDFWANELVIWSKKVAFDGVWYDMSEVSSFCVGSCGTGNLTLNPAHPSFLLPGEPGDIIYDY
PEAFNITNATEAASASAGASSQAAATATTTSTSVSYLRTTPTPGVRNVEHPPYVINHDQEGHD
LSVHAVSPNATHVDGVEEYDVHGLYGHQGLNATYQGLLEVWSHKRRPFIIGRSTFAGSGKWAG
HWGGDNYSKWWSMYYSISQALSFSLFGIPMFGADTCGFNGNSDEELCNRWMQLSAFFPFYRNH
NELSTIPQEPYRWASVIEATKSAMRIRYAILPYFYTLFDLAHTTGSTVMRALSWEFPNDPTLA
AVETQFMVGPAIMVVPVLEPLVNTVKGVFPGVGHGEVWYDWYTQAAVDAKPGVNTTISAPLGH
IPVYVRGGNILPMQEPALTTREARQTPWALLAALGSNGTASGQLYLDDGESIYPNATLHVDFT
ASRSSLRSSAQGRWKERNPLANVTVLGVNKEPSAVTLNGQAVFPGSVTYNSTSQVLFVGGLQN
LTKGGAWAENWVLEW

E2 GH31 α-glucosidase (SEQ ID NO: 2):

AAIVRRNGASPSCPGYKASNVKTVDGEIVSADLNLAGPACNVYGTDLDDLKLQVEYQSEQRLH
VKIYDAAEQVYQVPTAVLPRPSSANIPPAKSDLKFSMTNDPFSFTIKRRSNGEILFDTSGHPL
IFESQYLGLRTKLPDSPNIYGLGEHTGSFRLPTKNYTRTLWSRDAYGTPKDTNLYGNHPVYFD
YRGSNGTHGVFLLNSNGMDVDIDVDSDGQYLQYNTLGGVLDFYFLSGPDPKAVATQYAETVGK
PVMMPYWGFGFHNCRYGYQDIYEVAEIIANYSAANIPLETQWTDIDYMDLRKVFTLDPYRYPL
KLVQEVVSYLHKHNQHYIMMVDPAVAYQNYSAFNNGVAADAFLKFSNGSIYQGVVWPGPTAFP
DWFAPQTQEFWNSEFSTFFDPAHGVDIDALWIDMNEASNFCDFPCSNPAAYAAANGDPPTPPP
VRLSPPRPIPGFGPDFQPTCVATVSFDCDAQTYFGENILILGNSTTLGAGDVHMAPVMSANNY
PIWQLTVQMPPNGTFSYQYVRKESDGSYIYEQTNRTVTTGDCTSGTLKVSDTITTSSGPHKRS
ELRPLVRSPFPAEDLTRRQSGSMLGLPNRNLLNPPYTIHNAAGNLSEKTINTDLIHAGGYAEY
DTHNLYGTMMSATSREAMLNRRPAVRPLVITRSTFAGAGRQVGHWLGDNFADWDHYRWTIAEL
QEFAALFQIPMVGSDICGYDGNTTDNLCSRWVFLGAFSPFFRDHSDNQSPPHELYRTPQIAAA
ARAAIDIRYRLLDYAYTVLWTQTQTGAPMLNPMFFEYPADSNTADLQYQFFWGDSIMVAPVTD
NDSTTVNVYFPKDQFYDFYTGAPVSGEGNTVTLTDVGFDTIPLYFKGGSIVPMRVRSANTTAE
LRQQDFVVVIAPDSHGDATGQLYLDDGESINQPHTSEIQFSYRGGHFSMTGKFDYDPGNVVIS
QITLLGADGAGKGGSYNSTTKVATYKVNAKLTGKFEASLH

E3 GH31 α-glucosidase (SEQ ID NO: 3), also referred to, herein, as PvaGlu1:

AAISPAATSSTASWGPVFTVPASADEGAQLIANINDPQSVNAQTVCPGYVASNVQNNEFGFTA
TLNLAGKACNVYGTDVDSLNLTVQYQASDRLNINIGPAHVDASNESWYILSDDLVYKPTVDGT
ASISQSDLLVSWSNEPSFNFKVIRKANKDVLFNTEGTVLVYENQFIEFVSALPENYNLYGLGE
RIHGLRLGNNFTATTYAADAADPIDANIYGTHPFYLDTRYYEVDSKQGTYTLLTTNETDQSKE
YTSFSHGVFLRNAHGQEVLLRPEGITWRTLGGSIDLYFYSGPTQADVTRSYQTSTVGLPTMQQ

YYTFGYHQCRWGYQNWSVMADVVSSFAKFQIPLETIWSDIDYMNAYRDFENDPIRFSYSEGAE
FLGQLHENGQHWVPIVDSAIYIPNSENASDAYDVYTRGEADGVWMTNPDGSLYIGAVWPGYTV
FPDWHNPKAHEFWSNEIATWHQKVAFDGIWIDMSEVSSFCVGSCGTGNLTLNPVHPSFLLPGE
PGAVIYDYPESFNVTNSTEAASASAASVSQAAATASASASTTTSYLRTTPTPGVRDVNHPPYV
INNVQPGHDLAVHAVSPNATHIDGVSEYDVHNLWGYQILNATYHGLLKVWEDKRPFIIGRSTF
AGSGKWAGHWGGDNTSLWAYMFFSIPQALSFSLFGIPMFGVDTCGFNGNSDEELCNRWMQLSA
FFPFYRNHNVLSAISQEPYVWASVIDASKAAMKIRYALLPYIYTLFYLAHTTGSTVMRAVSWE
FPNDPSLAAIDTQFLLGPSLMVVPVLEPQVDYVKGVFPGVGNGEVWYDWYTQSVFDAKPGVNT
TISAPLGHIPVFVRGGSILPMQEPALTTRDARKTPWALLTALGGNGTASGQLYIDDGESITPN
ATLNVDFVASNSNLVASPRGSWVEKNPLANVTVLGVPTAPSSVTFNGAAVPRASVAYNSTSKT
LFVGGLQDFTKTGAWADKWVLKW

E4 GH31 α-glucosidase (SEQ ID NO: 4):

AATQTSSSAYVQTTLESSVDVGANLIANIDDPEAINAQSACPGYRASNVQNTSRGWTATLKLA
GKACNVYGTDVESLNFTLEYLSSTRVNIQITPSHVDSSNASWYHLSEDVVPRPKADKNASAKD
SHFEVSWSNEPSFGFKVARKATGDVLFNTIGSKLVYENQFIEFVTALPDDYNLYGLGEHIQQL
RLLKNSTFTLYAADTGDPVDLNTYGSHAFYLDTRYYEVNDKGSHTLVSSDQATTSKNYVSYSH
GVFLRNAHGQEILLGTGKLTWRTIGGSIDLTLYAGPTQTEVTKDYQLSTIGLPAMQQYFTFGY
HQCRWGYTNWSEVEDVVANFQKFEIPLENIWNDIDYMHGYRDFDNDQNRYSYEEGAVFLEKLH
KAGIHYIPIVDSALYIPDPNNASDAYDTYTRGAELDVFLKNPDGSTYIGAVWPGYTVFADWHH
PKAGDFWANELVTWHERVAFDGIWIDMNEVSSFCVGSCGSGKLSQNPVHPPFSLPGEPGNIIY
DYPEGFNATNSTEAASASAASASQASAAAATGQSAATTTTPYLRTTPTPGVRDVNHPPYVINH
AQTGHDLAVHAVSPNATHSDGVQEYDVHSLYGHSIIRATYEGLLKVFPEKRPFIIGRSTFAGT
GKWAGHWGGDNNSKWSYMFWSIPQALQFSLFGVPMFGVDTCGFNGNTDEELCNRWMQLSAFFP
FYRNHNVLSAISQEPYRWASVAEASKAAMKIRYAILPYMYTLFQQAHTTGSTVMRALAWEFPN
DPSLAAVDTQFLLGPSIMVVPVLAPQATSVKGVFPGIKQGEVWYDWYTQTAVDAQPHVNTTIA
APLGHIPVFVRGDSVLPMQEPALTTRDARNTPWTILAALGDKGTASGELYLDDGESLEPNATL
TVTFKATKSSLSAEPRGNWQEKNALGNVNVLGVAHKPNGVTLNGKAVPAASVHYNSTSQVLSV
TDLQKMTSKGAFANNWVLKW

Additional α-glucosidase include but are not limited to those having at least 70%, at least 80%, at least 90%, at least 95%, at least 97%, or even at least 99% amino acid sequence identity to SEQ ID NO: 1, SEQ ID NO: 2, SEQ ID NO: 3, and/or SEQ ID NO: 4.

In some embodiments, the GH31 α-glucosidase is added to corn fiber in an amount of less than 100, less than 70, less than 50, less than 40, less than 30, less than 25, or even less than 20 μg enzyme per g fiber. In some embodiments, the increase in glucose release from corn fiber is at least 1%, at least 2%, at least 5%, at least 10%, at least 20%, at least 30%, at least 40%, at least 50%, at least 60%, or even at least 70%, compared to the amount of glucose released under equivalent conditions in the absence of α-glucosidase. In some embodiments, the increase in ethanol obtained from corn fiber is at least 0.1%, at least 0.2%, at least 0.5%, at least 1.0%, or even at least 2.0% compared to the amount of produced under equivalent conditions in the absence of α-glucosidase. The absence of α-glucosidase encompasses conditions where a minor amount of background α-glucosidase may be present, *i.e.,* less than 5, less than 3, less than 2, or even less than 1 μg enzyme per g fiber.

## IV. Corn fiber materials from predominantly corn kernel feedstocks

The present methods involve the addition of GH31 α-glucosidase to corn fiber materials derived from a predominantly corn kernel feedstock (at least 80% corn kernels) that has been pretreated in preparation for enzymatic digestion.

In some embodiments, the methods relate to producing additional ethanol from a stillage by-product of an ethanol production facility that utilizes predominantly corn kernel feedstock (at least 80% corn kernels). Whole stillage, or simply stillage, refers to the solid mixture that remains after distilling ethanol and other low molecular components from a fermentation mixture (or beer). Typically, whole stillage falls to the bottom of distillation columns and is eventually transferred to one or more holding tanks to make room for a fresh batch of beer. Whole stillage may then be processed, *e.g.,* via centrifugation, to separate it into mostly-liquid thin stillage and wet distillers' grain (or wet cake). Wet cake may be sold as animal feed directly or dried to produce distillers' dry grain (DDG). Syrup obtained from the drying the thin stillage may be added to the DDG to produce DDG with solubles (DDGS), which is a preferred form of animal feed.

The present compositions and methods are directed at using whole stillage for the production of additional fermentable sugars. Derivatives of whole stillage, such as thin stillage, wet cake, DDG, and DDGS may also be used, although the additional processing steps are deemed unnecessary.

An exemplary process for the use of whole stillage for the production of fermentable sugars is described in US Patent No. 8, 633,033 (Assigned to Quad County Com Processors, Holstein, Iowa, USA). However, the importance and commercial value of using α-glucosidase to release fermentable carbohydrates from pretreated corn fiber in a starch-hydrolysis process are previously undescribed.

In other embodiments, the present methods relate to producing additional ethanol from corn fiber materials that are mechanically separated from carbohydrate material in a predominantly corn kernel feedstock (at least 80% corn kernels) at the beginning of a com starch production process. The corn fibers are typically separated mechanically, primarily to remove unwanted fiber from carbohydrate portion of the feedstock, which fibers can lead to fouling and clogging equipment and increase the demand for material handling. Following chemical pretreatment of the fibers to render them amenable to enzymatic digestion, the fibers are subsequently treated with a GH31 α-glucosidase.

In some embodiments, the amount of fiber present in the corn fiber materials derived from a predominantly corn kernel feedstock is less than about 30%, less than about 25%, less than about 20%, less than about 15%, less than about 12%, or even less than about 10% of the total dry weight of the substrate. Exemplary amounts are about 1-30%, about 1-25%, about 1-20%, about 1-15%, and about 1-12%.

## V. Chemical, thermal, or mechanical pretreatment

The present methods require some form of chemical, thermal, or mechanical pretreatment, or combination of, the corn fibers to prepare them for enzymatic digestion. Exemplary treatments are with dilute acid, optionally with heat and pressure, or more concentrated acid, typically at lower temperatures and pressures. Alternative treatments are with dilute ammonia. The optimum pH for α-glucosidase treatment largely depend on the optimum pH for the enzyme and is not believed to be critical.

In another embodiment, heating of the fiber substrate is performed wherein high pressure steam is injected into the substrate mixture to increase its temperature to about 215-300°F (*i.e.,* about 102-150°C), preferably in a container or vessel able to maintain a pressure above the vapor pressure of water in order to avoid boiling the sample. Heating by steam injection is beneficial because it aids in disruption of the fiber structure to improve subsequent enzymatic hydrolysis.

In another embodiment, mechanical pretreatment is used to produce uniform particle size of less than about 1,600 microns (μm). In some embodiments, at least 95% of the pretreated biomass particles are mechanically treated to have a particle size of from about 100 microns to about 800 microns, or a particle size from about 100 microns to about 500 microns. Such pretreated biomass particles can be generated by, for example, using a hammer mill or a colloid mill.

## EXAMPLES

### EXAMPLE 1. Cloning of *Paecilomyces variotii* α-glucosidase (PvaGlul)

*Paecilomyces variotii* NRRL1115 strain was selected as a potential source for various enzymes, useful for industrial applications. The entire genome of the *P. variotii* NRRL1115 strain was sequenced and the nucleotide sequence of a putative α-glucosidase, designated "PvaGlu1" was identified by sequence identity. The gene encoding PvaGlul is set forth as SEQ ID NO:5:

ATGGGCGGCTTCACCCACTACATGCTCGCTTCCGCTTGGCTGCCTCTGACCCTGGGCGCCGCC
ATCTCTCCCGCTGCTACCAGCAGCACCGCTTCGTGGGGCCCCGTTTTCACCGTCCCCGCCTCC
GCCGACGAGGGCGCTCAGCTCATTGCCAACATTAACGACCCCCAGTCTGTTAACGCCCAGACC
GTTTGCCCCGGCTACGTTGCTTCTAACGTCCAGAACAACGAGTTCGGCTTCACCGCCACCCTG
AACCTCGCCGGCAAGGCTTGCAACGTTTACGGCACCGACGTCGATAGCCTGAACCTGACCGTC
CAGTACCAGGCGAGTGACCGACTGAACATTAACATTGGCCCCGCCCACGTTGACGCCTCCAAC
GAGTCTTGGTACATTCTGAGCGACGACCTGGTTTACAAGCCTACCGTGGACGGCACCGCTTCT
ATCTCTCAGTCTGACCTGCTGGTTAGCTGGTCTAACGAGCCTTCTTTCAACTTCAAGGTTATT
CGTAAGGCTAACAAGGACGTCCTGTTCAACACCGAGGGCACCGTCCTCGTTTACGAGAACCAG
TTCATCGAGTTCGTCAGCGCTCTCCCTGAGAACTACAACCTGTACGGCCTGGGCGAGCGCATT
CACGGCCTGCGACTCGGCAACAACTTCACCGCCACCACCTACGCCGCCGACGCCGCCGACCCC
ATCGACGCTAACATATATGGCACCCACCCTTTCTACCTGGACACCCGATACTACGAGGTTGAC
TCCAAGCAGGGCACCTACACCCTGCTGACCACCAACGAGACCGACCAGTCTAAGGAATACACC
TCTTTCTCTCACGGCGTTTTCCTCCGAAACGCTCACGGCCAGGAAGTTCTGCTGCGCCCTGAG
GGCATCACCTGGCGAACCCTGGGCGGCAGCATTGACCTGTACTTCTACTCCGGCCCTACCCAG
GCTGACGTCACCCGATCTTACCAGACCAGCACCGTTGGCCTGCCTACCATGCAGCAGTACTAC
ACCTTCGGCTACCACCAGTGCCGATGGGGCTACCAGAACTGGTCTGTCATGGCTGACGTTGTT
AGCTCTTTCGCCAAGTTCCAGATTCCTCTGGAGACCATTTGGAGCGACATCGACTACATGAAC
GCTTACCGAGACTTCGAGAACGACCCTATCCGATTCTCTTACTCTGAGGGCGCTGAGTTCCTC
GGCCAGCTGCACGAGAACGGCCAGCACTGGGTTCCTATTGTTGACTCCGCCATCTACATCCCT
AACAGCGAGAACGCCAGCGACGCTTACGACGTTTACACCCGAGGCGAGGCCGACGGCGTTTGG
ATGACCAACCCCGACGGCAGCCTGTACATTGGCGCCGTTTGGCCCGGCTACACCGTTTTCCCC
GACTGGCACAACCCTAAGGCTCACGAGTTCTGGTCTAACGAGATTGCTACCTGGCACCAGAAG
GTCGCTTTCGACGGCATTTGGATTGACATGTCTGAGGTCAGCTCTTTCTGCGTTGGCTCTTGC
GGCACCGGCAACCTGACCCTCAACCCCGTCCACCCTTCTTTCCTGCTCCCCGGCGAGCCCGGC
GCTGTTATATATGACTACCCTGAGTCTTTCAACGTCACCAACTCTACCGAGGCTGCTTCTGCT
TCAGCCGCATCGGTCTCCCAGGCTGCTGCTACCGCTTCTGCCTCTGCCTCAACCACCACCTCT
TACCTGCGAACCACCCCTACCCCCGGCGTCCGAGACGTTAACCACCCTCCTTACGTTATTAAC
AACGTCCAGCCTGGCCACGACCTCGCCGTCCACGCCGTTAGCCCCAACGCTACCCACATTGAC
GGCGTTAGCGAGTACGACGTCCACAACCTGTGGGGCTACCAGATTCTGAACGCTACCTACCAC
GGCCTCCTCAAGGTTTGGGAGGACAAGCGCCCTTTCATTATTGGCCGAAGCACCTTCGCTGGC
AGCGGCAAGTGGGCTGGCCACTGGGGCGGCGACAACACCTCTCTGTGGGCTTACATGTTCTTC
AGCATTCCTCAGGCTCTGTCTTTCAGCCTGTTCGGCATCCCCATGTTCGGCGTTGACACCTGC
GGCTTCAACGGCAACTCCGACGAAGAACTGTGCAACCGATGGATGCAGCTGTCTGCTTTCTTC

CCTTTCTACCGAAACCACAACGTCCTCAGCGCTATTAGCCAAGAACCTTACGTTTGGGCCTCC
GTCATTGACGCTTCTAAGGCCGCCATGAAGATTCGATACGCTCTGCTGCCTTACATATACACC
CTGTTCTACCTCGCTCACACCACCGGCAGCACCGTCATGCGAGCTGTCTCTTGGGAGTTCCCT
AACGACCCTAGCCTCGCCGCCATTGACACCCAGTTCCTCCTGGGCCCTAGCCTCATGGTTGTC
CCCGTCCTGGAGCCTCAGGTTGACTACGTTAAGGGCGTTTTCCCCGGCGTTGGCAACGGCGAG
GTTTGGTACGACTGGTACACCCAGTCTGTTTTCGACGCCAAGCCCGGCGTTAACACCACCATC
AGCGCTCCTCTCGGCCACATCCCCGTTTTCGTCCGAGGCGGCAGCATTCTGCCTATGCAAGAG
CCCGCTCTGACCACCCGCGACGCTCGAAAGACCCCTTGGGCTCTCCTGACCGCTCTGGGCGGC
AACGGCACCGCTTCTGGCCAGCTGTACATCGACGACGGCGAGTCTATTACCCCTAACGCCACC
CTGAACGTGGACTTCGTTGCTTCCAACTCTAACCTCGTTGCGTCACCTCGGGGCTCTTGGGTT
GAGAAGAACCCTCTCGCTAACGTTACCGTCCTCGGCGTCCCTACCGCTCCTAGCTCCGTTACC
TTCAACGGCGCCGCTGTTCCTCGCGCTTCCGTCGCTTACAACTCTACCTCCAAGACCCTGTTC
GTTGGCGGCCTGCAAGACTTCACCAAGACCGGCGCTTGGGCTGACAAGTGGGTTCTCAAGTGG

The amino acid sequence of the PvaGlul precursor protein is set forth as SEQ ID NO: 6. The predicted native signal peptide is shown in italics and underlined:

<u>*MGGFTHYMLASAWLPLTLG*</u>AAISPAATSSTASWGPVFTVPASADEGAQLIANINDPQSVNAQT
VCPGYVASNVQNNEFGFTATLNLAGKACNVYGTDVDSLNLTVQYQASDRLNINIGPAHVDASN
ESWYILSDDLVYKPTVDGTASISQSDLLVSWSNEPSFNFKVIRKANKDVLFNTEGTVLVYENQ
FIEFVSALPENYNLYGLGERIHGLRLGNNFTATTYAADAADPIDANIYGTHPFYLDTRYYEVD
SKQGTYTLLTTNETDQSKEYTSFSHGVFLRNAHGQEVLLRPEGITWRTLGGSIDLYFYSGPTQ
ADVTRSYQTSTVGLPTMQQYYTFGYHQCRWGYQNWSVMADVVSSFAKFQIPLETIWSDIDYMN
AYRDFENDPIRFSYSEGAEFLGQLHENGQHWVPIVDSAIYIPNSENASDAYDVYTRGEADGVW
MTNPDGSLYIGAVWPGYTVFPDWHNPKAHEFWSNEIATWHQKVAFDGIWIDMSEVSSFCVGSC
GTGNLTLNPVHPSFLLPGEPGAVIYDYPESFNVTNSTEAASASAASVSQAAATASASASTTTS
YLRTTPTPGVRDVNHPPYVINNVQPGHDLAVHAVSPNATHIDGVSEYDVHNLWGYQILNATYH
GLLKVWEDKRPFIIGRSTFAGSGKWAGHWGGDNTSLWAYMFFSIPQALSFSLFGIPMFGVDTC
GFNGNSDEELCNRWMQLSAFFPFYRNHNVLSAISQEPYVWASVIDASKAAMKIRYALLPYIYT
LFYLAHTTGSTVMRAVSWEFPNDPSLAAIDTQFLLGPSLMVVPVLEPQVDYVKGVFPGVGNGE
VWYDWYTQSVFDAKPGVNTTISAPLGHIPVFVRGGSILPMQEPALTTRDARKTPWALLTALGG
NGTASGQLYIDDGESITPNATLNVDFVASNSNLVASPRGSWVEKNPLANVTVLGVPTAPSSVT
FNGAAVPRASVAYNSTSKTLFVGGLQDFTKTGAWADKWVLKW

The amino acid sequence of the mature form of PvaGlul (also referred to herein as E3 GH31 α-glucosidase) is set forth as SEQ ID NO:3, which is shown, above.

### Example 2. Expression and purification of PvaGlu1

The nucleotide sequence of a synthetic gene encoding PvaGlul optimized for expression in *Trichoderma reesei* is set forth as SEQ ID NO. 7:

ATGGGCGGCTTCACCCACTACATGCTCGCTTCCGCTTGGCTGCCTCTGACCCTGGGCGCCGCC
ATCTCTCCCGCTGCTACCAGCAGCACCGCTTCGTGGGGCCCCGTTTTCACCGTCCCCGCCTCC
GCCGACGAGGGCGCTCAGCTCATTGCCAACATTAACGACCCCCAGTCTGTTAACGCCCAGACC
GTTTGCCCCGGCTACGTTGCTTCTAACGTCCAGAACAACGAGTTCGGCTTCACCGCCACCCTG

AACCTCGCCGGCAAGGCTTGCAACGTTTACGGCACCGACGTCGATAGCCTGAACCTGACCGTC
CAGTACCAGGCGAGTGACCGACTGAACATTAACATTGGCCCCGCCCACGTTGACGCCTCCAAC
GAGTCTTGGTACATTCTGAGCGACGACCTGGTTTACAAGCCTACCGTGGACGGCACCGCTTCT
ATCTCTCAGTCTGACCTGCTGGTTAGCTGGTCTAACGAGCCTTCTTTCAACTTCAAGGTTATT
CGTAAGGCTAACAAGGACGTCCTGTTCAACACCGAGGGCACCGTCCTCGTTTACGAGAACCAG
TTCATCGAGTTCGTCAGCGCTCTCCCTGAGAACTACAACCTGTACGGCCTGGGCGAGCGCATT
CACGGCCTGCGACTCGGCAACAACTTCACCGCCACCACCTACGCCGCCGACGCCGCCGACCCC
ATCGACGCTAACATATATGGCACCCACCCTTTCTACCTGGACACCCGATACTACGAGGTTGAC
TCCAAGCAGGGCACCTACACCCTGCTGACCACCAACGAGACCGACCAGTCTAAGGAATACACC
TCTTTCTCTCACGGCGTTTTCCTCCGAAACGCTCACGGCCAGGAAGTTCTGCTGCGCCCTGAG
GGCATCACCTGGCGAACCCTGGGCGGCAGCATTGACCTGTACTTCTACTCCGGCCCTACCCAG
GCTGACGTCACCCGATCTTACCAGACCAGCACCGTTGGCCTGCCTACCATGCAGCAGTACTAC
ACCTTCGGCTACCACCAGTGCCGATGGGGCTACCAGAACTGGTCTGTCATGGCTGACGTTGTT
AGCTCTTTCGCCAAGTTCCAGATTCCTCTGGAGACCATTTGGAGCGACATCGACTACATGAAC
GCTTACCGAGACTTCGAGAACGACCCTATCCGATTCTCTTACTCTGAGGGCGCTGAGTTCCTC
GGCCAGCTGCACGAGAACGGCCAGCACTGGGTTCCTATTGTTGACTCCGCCATCTACATCCCT
AACAGCGAGAACGCCAGCGACGCTTACGACGTTTACACCCGAGGCGAGGCCGACGGCGTTTGG
ATGACCAACCCCGACGGCAGCCTGTACATTGGCGCCGTTTGGCCCGGCTACACCGTTTTCCCC
GACTGGCACAACCCTAAGGCTCACGAGTTCTGGTCTAACGAGATTGCTACCTGGCACCAGAAG
GTCGCTTTCGACGGCATTTGGATTGACATGTCTGAGGTCAGCTCTTTCTGCGTTGGCTCTTGC
GGCACCGGCAACCTGACCCTCAACCCCGTCCACCCTTCTTTCCTGCTCCCCGGCGAGCCCGGC
GCTGTTATATATGACTACCCTGAGTCTTTCAACGTCACCAACTCTACCGAGGCTGCTTCTGCT
TCAGCCGCATCGGTCTCCCAGGCTGCTGCTACCGCTTCTGCCTCTGCCTCAACCACCACCTCT
TACCTGCGAACCACCCCTACCCCCGGCGTCCGAGACGTTAACCACCCTCCTTACGTTATTAAC
AACGTCCAGCCTGGCCACGACCTCGCCGTCCACGCCGTTAGCCCCAACGCTACCCACATTGAC
GGCGTTAGCGAGTACGACGTCCACAACCTGTGGGGCTACCAGATTCTGAACGCTACCTACCAC
GGCCTCCTCAAGGTTTGGGAGGACAAGCGCCCTTTCATTATTGGCCGAAGCACCTTCGCTGGC
AGCGGCAAGTGGGCTGGCCACTGGGGCGGCGACAACACCTCTCTGTGGGCTTACATGTTCTTC
AGCATTCCTCAGGCTCTGTCTTTCAGCCTGTTCGGCATCCCCATGTTCGGCGTTGACACCTGC
GGCTTCAACGGCAACTCCGACGAAGAACTGTGCAACCGATGGATGCAGCTGTCTGCTTTCTTC
CCTTTCTACCGAAACCACAACGTCCTCAGCGCTATTAGCCAAGAACCTTACGTTTGGGCCTCC
GTCATTGACGCTTCTAAGGCCGCCATGAAGATTCGATACGCTCTGCTGCCTTACATATACACC
CTGTTCTACCTCGCTCACACCACCGGCAGCACCGTCATGCGAGCTGTCTCTTGGGAGTTCCCT
AACGACCCTAGCCTCGCCGCCATTGACACCCAGTTCCTCCTGGGCCCTAGCCTCATGGTTGTC
CCCGTCCTGGAGCCTCAGGTTGACTACGTTAAGGGCGTTTTCCCCGGCGTTGGCAACGGCGAG
GTTTGGTACGACTGGTACACCCAGTCTGTTTTCGACGCCAAGCCCGGCGTTAACACCACCATC
AGCGCTCCTCTCGGCCACATCCCCGTTTTCGTCCGAGGCGGCAGCATTCTGCCTATGCAAGAG
CCCGCTCTGACCACCCGCGACGCTCGAAAGACCCCTTGGGCTCTCCTGACCGCTCTGGGCGGC
AACGGCACCGCTTCTGGCCAGCTGTACATCGACGACGGCGAGTCTATTACCCCTAACGCCACC
CTGAACGTGGACTTCGTTGCTTCCAACTCTAACCTCGTTGCGTCACCTCGGGGCTCTTGGGTT
GAGAAGAACCCTCTCGCTAACGTTACCGTCCTCGGCGTCCCTACCGCTCCTAGCTCCGTTACC
TTCAACGGCGCCGCTGTTCCTCGCGCTTCCGTCGCTTACAACTCTACCTCCAAGACCCTGTTC
GTTGGCGGCCTGCAAGACTTCACCAAGACCGGCGCTTGGGCTGACAAGTGGGTTCTCAAGTGG

The foregoing gene was inserted into the pTTT expression vector (described in WO2015/017256), resulting in plasmid pTTT-PvaGlu1 (Figure 1). pTTT-PvaGlu1 was transformed into a quad-deleted *Trichoderma reesei* strain (described in WO 05/001036) using protoplast transformation (Te'o et al. (2002) J. Microbiol. Methods 51:393-99). Transformants were selected and grown using the methods described in WO 2016/138315. Supernatants from these cultures were used to confirm the protein expression by SDS-PAGE analysis and in assays to measure enzyme activity.

A seed culture of the transformed cells mentioned was subsequently grown in a 2.8 L fermenter in defined medium. Fermentation broth was sampled at elapsed times of 42 hours, 65 hours, and 95 hours for SDS-PAGE analysis and measurements of dry cell weight, residual glucose and extracellular protein concentration. Following centrifugation, filtration and concentration, 500 mL of concentrated sample having a protein concentration of 10.7 g/L was obtained. PvaGluI was purified via hydrophobic interaction chromatography (HIC) followed by ammonium sulfate precipitation, and then stored at -20°C until needed.

**Example 3. Substrate specificity of PvaGluI**

Substrate specificity of PvaGluI was assayed based on the release of glucose following incubation with isomaltose, maltose, trehalose, panose, maltulose, sucrose, leucrose, nigerose, kojibiose, maltotriose or maltoheptaose. The rate of glucose release was measured using a coupled glucose oxidase/peroxidase (GOX/HRP) method (Ngo, T.T. and Lenhoff, H.M. (1980) Anal. Biochem. 105:389-397). Glucose was quantified as the rate of oxidation of 2,2'-azino-bis 3-ethylbenzothiazoline-6-sulfonic acid (ABTS) by peroxide which was generated from coupled GOX/HRP enzymes reacted with glucose.

Substrate solutions were prepared by mixing 9 mL of each substrate mentioned above (1% in water, w/v), 1 mL of 0.5 M pH 5.0 sodium acetate buffer, and 40 $\mu$L of 0.5 M calcium chloride in a 15-mL conical tube. Coupled enzyme (GOX/HRP) solution with ABTS was prepared in 50 mM sodium acetate buffer (pH 5.0), with the final concentrations of 2.74 mg/mL ABTS, 0.1 U/mL HRP, and 1 U/mL GOX.

Serial dilutions of $\alpha$-glucosidase samples and glucose standard were prepared in Milli Q water. Each $\alpha$-glucosidase sample (10 $\mu$L) was transferred into a new microtiter plate (Corning 3641) containing 90 $\mu$L of substrate solution preincubated at 50 °C for 5 min at 600 rpm. The reactions were carried out at 50°C for 10 min (for isomaltose, maltose, panose, maltulose, nigerose, and kojibiose) and for 60 min (for sucrose and leucrose) with shaking

(600 rpm) in a thermomixer (Eppendorf). 10 $\mu$L of reaction mixtures as well as 10 $\mu$L of serial dilutions of glucose standard were quickly transferred to new microtiter plates (Corning 3641), followed by the addition of 90 $\mu$L of ABTS/GOX/HRP solution. The microtiter plates containing the reaction mixture were immediately measured at 405 nm at 11 seconds intervals for 5 min on SoftMax Pro plate reader (Molecular Device). The output was the reaction rate, Vo, for each enzyme concentration. Linear regression was used to determine the slope of the plot Vo versus enzyme dose. The specific activity of $\alpha$-glucosidase was calculated based on the glucose standard curve using the following equation:

$$\text{Specific Activity (Unit/mg)} = \text{Slope (enzyme)} / \text{slope (std)} \times 1000 \quad (1),$$

where 1 Unit = 1 $\mu$mol glucose /min.

Except for isomaltose and maltose, the value of the reaction rate with enzyme dosage at 5 ppm was directly used to indicate the enzyme activity on other substrates listed. Using the method mentioned above, substrate specificity of PvaGluI was determined and compared with the benchmarks, oligo-1,6-glucosidase (a product purchased from Megazyme) and purified TrTG (transglucosidase from *Trichoderma reesei*) as shown in Table 1. PvaGluI showed broader substrate specificity than the other enzymes, with high activity towards isomaltose, maltose, nigerose, and kojibiose and moderate activity towards panose, maltotriose and maltoheptaose.

**Table 1.** Substrate specificity of purified PvaGlu1compared with Oligo-1,6-glucosidase and TrTG.

| Enzyme | Isomaltose (U/mg) | Maltose (U/mg) | Trehalose (5 ppm) | Leucrose (5 ppm) | Sucrose (5 ppm) | Maltulose (5 ppm) | Panose (5 ppm) | Maltotriose (5 ppm) | Maltoheptaose (5 ppm) | Kojibiose (5 ppm) | Nigerose (5 ppm) |
|---|---|---|---|---|---|---|---|---|---|---|---|
| oligo-1,6-gluc. | 118.2 | 0.0 | 3.3 | 0.8 | 1.2 | 7.1 | 20.3 | 2.2 | 1.2 | 5.1 | 27.3 |
| TrTG | 203.1 | 267.9 | 6.5 | 12.7 | 0.4 | 2.1 | 72.7 | 41.5 | 38.2 | 58.9 | 118.4 |
| PvaGlu1 | 398.2 | 230.8 | 2.4 | 16.9 | 0.7 | 4.2 | 64.9 | 42.4 | 64.3 | 135.1 | 188.6 |

**Example 4. pH profile of PvaGlul**

The effect of pH (from 3.0 to 10.0) on α-glucosidase activity of PvaGlul was monitored using isomaltose as a substrate. Buffer working solutions consisted of the combination of glycine/sodium acetate/HEPES (250 mM), with pH varying from 3.0 to 10.0. Substrate solutions were prepared by mixing isomaltose (1% in water, w/v) with 250 mM buffer solution at a ratio of 9:1, containing calcium chloride at a final concentration of 2 mM. Enzyme working solutions were prepared in water at a certain dose (showing signal within linear range as per dose response curve). All the incubations were carried out at 50°C for 10 min following the same protocol as described in Example 3 for activity of α-glucosidase towards isomaltose. Enzyme activity at each pH was reported as relative activity compared to enzyme activity at optimum pH. The results are shown in Table 2. PvaGlul was found to have an optimum pH at 5.0 and retain greater than 70% of maximum activity between 3.4 and 6.6.

**Table 2.** pH profile for PvaGlu1.

| pH | Relative activity (%) |
|---|---|
| 3 | 55 |
| 4 | 92 |
| 5 | 100 |
| 6 | 90 |
| 7 | 53 |
| 8 | 13 |
| 9 | 2 |
| 10 | 0 |

**Example 5. Temperature profile of PvaGlu1**

The effect of temperature (from 40 °C to 85 °C) on α-glucosidase activity of PvaGlul was monitored using isomaltose as a substrate. Substrate solutions were prepared by mixing 9 mL of isomaltose (1% in water, w/v), 1 mL of 0.5 M pH 5.0 sodium acetate buffer, and 40 μL of 0.5 M calcium chloride in a 15-mL conical tube. Enzyme working solutions were prepared in water at a certain dose (showing signal within linear range as per dose response curve). Incubations were performed at temperatures from 40 °C to 85 °C for 10 min following the same protocol as described in Example 3 for activity of α-glucosidase towards isomaltose. Activity at each temperature was reported as relative activity compared to enzyme activity at optimum temperature. The results are shown in Table 3. PvaGlul was found to have an optimum temperature at 55-60°C and was able to keep higher than 70% of maximum activity between 49°C and 68 °C.

**Table 3.** Temperature profile of PvaGluI

| Temp. (°C) | Relative activity (%) |
|---|---|
| 40 | 43 |
| 44.7 | 63 |
| 49.4 | 73 |
| 55 | 100 |
| 59.7 | 100 |
| 65 | 95 |
| 69.2 | 60 |
| 74.6 | 22 |
| 80 | 20 |
| 85 | 16 |

**Example 6. Glucose release from corn fiber in the presence of α-glucosidase**

Whole stillage, the by-product of a starch ethanol fermentation and distillation, was prepared for a secondary saccharification by treatment with acid (see, *e.g.,* U.S. Patent No. 8,633,003) and addition of enzymes. Specifically, about 0.4 grams of material containing approximately 20% dry solids was placed in a reaction vessel along with the commercial enzymes ACCELERASE TRIO™ and STARGEN™ 002 (DuPont Industrial Biosciences).

ACCELERASE TRIO™ includes several enzymatic activities, including 2000-2600 CMC U/g endoglucanase activity, >3000 ABX U/g xylanase activity and >2000 pNPG U/g β-glucosidase activity. One carboxy methyl cellulose (CMC) unit is defined as the amount of activity required to liberate 1 μmol of reducing sugars (expressed as glucose equivalents) in one minute at 50°C (122°F) at pH 4.8. One ABX unit is defined as the amount of enzyme required to generate 1 μmol of xylose reducing sugar equivalents per minute at 50°C (122°F) and pH 5.3. One pNPG unit denotes 1 μmol of nitro-phenol liberated from paranitrophenyl-B-D-glucopyranoside per minute at 50°C (122°F) and pH 4.8. ACCELERASE TRIO™ has negligible, if any, α-glucosidase activity. ACCELERASE TRIO™ was dosed at 0.07% wt/vol relative to stillage.

STARGEN™ 002 enzyme contains the α-amylase from *Aspergillus kawachi* and the glucoamylase from *Trichoderma reesei.* One glucoamylase Unit (GAU) is the amount of enzyme that will liberate one gram of reducing sugars calculated as glucose per hour from soluble starch substrate under the conditions of the assay. STARGEN™ 002 has minimal α-glucosidase activity, which may be attributable to the small amount α-glucosidase activity associated with α-amylases. STARGEN™ 002 was dosed at 0.017 GAU/gram dry solid stillage.

To the above reactions several different concentrations of purified α-glucosidase was added and the reaction vessels placed in an incubator at 50°C for 18-24 hr with shaking, followed by termination by the addition of 0.2 M $H_2SO_4$. Following centrifugation, the supernatant was collected and analyzed by HPLC to measure the concentration of glucose. The name, Carbohydrate Active Enzyme (CAZy) classification, and SEQ ID NO of the enzymes tested is shown in Table 4, below. Note that E3 is PvaGluI described in Examples 1-5.

**Table 4.** α-glucosidases tested

| Name | CAZy classification | SEQ ID NO |
|---|---|---|
| E1 | GH31 | 1 |
| E2 | GH31 | 2 |
| E3 | GH31 | 3 |
| E4 | GH31 | 4 |
| D1 | GH97 | n/a |
| D2 | GH97 | n/a |
| D3 | GH97 | n/a |

The results are shown in Figure 2. The addition of both GH31 and GH97 α-glucosidases results in increased glucose release in a dose-dependent manner. However, while the increase observed with GH97 enzymes is only about 1%, the increase observed with GH31 enzymes is as much as 60-70%, with a significant amount of the increase being observed at below 50, and even below 25 μg enzyme per g dry solids.

## Claims

1. A method for increasing the production of fermentable sugars in an ethanol fermentation process, comprising: chemically-pretreating corn fiber material derived from a predominantly corn kernel feedstock containing at least 80% corn kernels; and contacting the pretreated corn fiber with α-glucosidase (EC 3.2.1.20) to release fermentable sugars from the corn fiber; wherein the contacting with α-glucosidase releases fermentable sugars from the corn fiber material derived from a predominantly corn kernel feedstock that otherwise would not be available for fermentation, wherein the α-glucosidase is a GH31 enzyme and wherein the contacting with α-glucosidase is performed in the presence of one or more additional enzymes.

2. The method of claim 1, wherein the corn fiber material is the stillage resulting from distillation of a fermentation product, or a derivative, thereof.

3. The method of claim 1, wherein the corn fiber material is produced by physically separating corn fiber from starch in a substrate for ethanol fermentation.

4. The method of claim 1, wherein the corn fiber material is exposed to a temperature of at least 80°C prior to the contacting with α-glucosidase.

5. The method of claim 1, wherein the corn fiber material is subjected to an alcohol distillation step prior to the contacting with α-glucosidase.

6. The method of claim 1, wherein the corn fiber material contains 1-12% corn fiber (wt/wt).

7. The method of any of the preceding claims, wherein the pretreatment is acid pretreatment or alkaline pretreatment.

8. The method of any of the preceding claims, wherein the α-glucosidase has at least 70% amino acid sequence identity to a polypeptide having an amino acid sequence selected from the group consisting of SEQ ID NO: 1, SEQ ID NO: 2, SEQ ID NO: 3 and SEQ ID NO: 4.

9. A method for increasing the production of fermentable sugars from chemically-pretreated stillage, comprising:

fermenting a predominantly corn kernel feedstock containing at least 80% corn kernels with an ethanolagen to produce ethanol;
evaporating at least a portion of the ethanol and recovering stillage;
chemically-pretreating the stillage;
contacting the stillage with α-glucosidase (EC 3.2.1.20) to release fermentable sugars;
fermenting the released fermentable sugars with an ethanolagen to produce additional ethanol; and recovering the additional ethanol;
wherein the contacting with α-glucosidase releases fermentable sugars from the corn fiber material derived from the predominantly corn kernel feedstock that otherwise would not be available for fermentation
wherein the α-glucosidase is a GH31 enzyme and wherein the contacting with α-glucosidase is performed in the presence of one or more additional enzymes.

10. The method of claim 9, wherein the pretreatment is acid pretreatment or alkaline pretreatment.

11. The method of any of claims 9-10, wherein the corn fiber material contains 1-12% corn fiber (wt/wt).

12. The method of any of claims 9-11, wherein the α-glucosidase has at least 70% amino acid sequence identity to a polypeptide having an amino acid sequence selected from the group consisting of SEQ ID NO: 1, SEQ ID NO: 2, SEQ ID NO: 3 and SEQ ID NO: 4.

13. A polypeptide having α-glucosidase activity, selected from the group consisting of:

(a) a polypeptide comprising an amino acid sequence having at least 97% identity to the polypeptide of SEQ ID NO: 3;
(b) a mature polypeptide produced by the processing of the polypeptide of SEQ ID NO: 6 by a signal peptidase or post translational modification during secretion from an expression host.

14. A polypeptide according to claim 13, wherein alternative (a) comprises an amino acid sequence having at least 99% identity to the polypeptide of SEQ ID NO: 3.

15. A polynucleotide comprising a nucleotide sequence that encodes the polypeptide of claim 13 or claim 14.

## Patentansprüche

1. Verfahren zum Erhöhen der Produktion von fermentierbaren Zuckern in einem Prozess zur Fermentation von Ethanol, umfassend: chemisches Vorbehandeln von Maisfasermaterial, das überwiegend aus einem Maiskorn-Ausgangsmaterial stammt, das mindestens 80% Maiskörner enthält; und Inkontaktbringen der vorbehandelten Maisfaser mit α-Glucosidase (EC 3.2.1.20), um aus der Maisfaser fermentierbaren Zucker freizusetzen; wobei das Inkontaktbringen mit α-Glucosidase fermentierbare Zucker aus dem Maiskorn-Fasermaterial freisetzt, das aus einem überwiegenden Maiskorn-Ausgangsmaterial stammt, das auf andere Weise zur Fermentation nicht verfügbar sein würde, wobei die α-Glucosidase ein GH31 Enzym ist und wobei das Inkontaktbringen mit α-Glucosidase in Gegenwart von einem oder mehreren zusätzlichen Enzymen ausgeführt wird.

2. Verfahren nach Anspruch 1, wobei das Maisfasermaterial die Schlempe ist, die aus Destillation eines Fermentationsprodukts resultiert, oder ein Derivats davon.

3. Verfahren nach Anspruch 1, wobei das Maisfasermaterial hergestellt wird durch physikalisches Trennen von Maisfaser von Stärke in einem Substrat zur Fermentation von Ethanol.

4. Verfahren nach Anspruch 1, wobei das Maisfasermaterial vor dem Inkontaktbringen mit α-Glucosidase einer Temperatur von mindestens 80 °C ausgesetzt wird.

5. Verfahren nach Anspruch 1, wobei das Maisfasermaterial vor dem Inkontaktbringen mit α-Glucosidase einem Schritt einer Alkoholdestillation unterworfen wird.

6. Verfahren nach Anspruch 1, wobei das Maisfasermaterial 1 bis 12% Maisfaser (Gew./Gew.) enthält.

7. Verfahren nach einem der vorhergehenden Ansprüche, wobei die Vorbehandlung eine saure Vorbehandlung oder basische Vorbehandlung ist.

8. Verfahren nach einem der vorhergehenden Ansprüche, wobei die α-Glucosidase mindestens eine Identität der Aminosäuresequenz von 70% zu einem Polypeptid mit einer Aminosäuresequenz aufweist, die ausgewählt ist aus der Gruppe bestehend aus SEQ ID NO: 1, SEQ ID NO: 2, SEQ ID NO: 3 und SEQ ID NO: 4.

9. Verfahren zum Erhöhen der Produktion von fermentierbaren Zuckern aus chemisch vorbehandelter Schlempe, umfassend:

Fermentieren eines überwiegenden Maiskorn-Ausgangsmaterials, das mindestens 80% Maiskörner enthält, mit einem Ethanolagen, um Ethanol zu erzeugen;
Verdampfen mindestens eines Teils des Ethanols und Gewinnen von Schlempe;
chemisches Vorbehandeln der Schlempe;
Inkontaktbringen der Schlempe mit α-Glucosidase (EC 3.2.1.20), um fermentierbare Zucker freizusetzen;
Fermentieren der freigesetzten Zucker mit einem Ethanolagen, um zusätzliches Ethanol zu erzeugen; und
Gewinnen des zusätzlichen Ethanols;
wobei das Inkontaktbringen mit α-Glucosidase fermentierbare Zucker aus dem Maisfasermaterial freisetzt, das überwiegend aus einem Maiskorn-Ausgangsmaterial stammt, das auf andere Weise zur Fermentation nicht verfügbar sein würde,
wobei die α-Glucosidase ein GH31 Enzym ist und wobei das Inkontaktbringen mit α-Glucosidase in Gegenwart von einem oder mehreren zusätzlichen Enzymen ausgeführt wird.

10. Verfahren nach Anspruch 9, wobei die Vorbehandlung eine saure Vorbehandlung oder basische Vorbehandlung ist.

11. Verfahren nach einem der Ansprüche 9 bis 10, wobei das Maisfasermaterial 1 bis 12% Maisfaser (Gew./Gew.) enthält.

12. Verfahren nach einem der Ansprüche 9 bis 11, wobei die α-Glucosidase mindestens eine Identität der Aminosäuresequenz von 70% zu einem Polypeptid mit einer Aminosäuresequenz aufweist, die ausgewählt ist aus der Gruppe bestehend aus SEQ ID NO: 1, SEQ ID NO: 2, SEQ ID NO: 3, und SEQ ID NO: 4.

13. Polypeptid, das eine α-Glucosidaseaktivität aufweist, ausgewählt aus der Gruppe bestehend aus:

(a) einem Polypeptid, umfassend eine Aminosäuresequenz das mindestens 97% Identität zu dem Polypeptid von SEQ ID NO:3 aufweist;
(b) einem reifen Polypeptid, das durch Verarbeitung des Polypeptids von SEQ ID NO: 6, durch eine Signalpeptidase- oder post-translationale Modifikation während der Sekretion von einem Expresssionwirt erzeugt wird.

14. Polypeptid nach Anspruch 13, wobei Alternative (a) eine Aminosäuresequenz umfasst, die mindestens 99% Indentität zu dem Polypeptid von SEQ ID NO:3 aufweist.

15. Polynukleotid, umfassend eine Nukleotidsequenz, die das Polypeptid nach Anspruch 13 oder Anspruch 14 kodiert.

## Revendications

1. Procédé pour augmenter la production de sucres fermentables dans un procédé de fermentation d'éthanol, comprenant: le prétraitement chimique d'une matière de fibre de maïs issue d'une matière première principalement de grains de maïs contenant au moins 80% de grains de maïs; et la mise en contact de la fibre de maïs prétraitée avec de la α-glucosidase (EC 3.2.1.20) pour libérer des sucres fermentables à partir de la fibre de maïs; dans lequel la mise en contact avec la α-glucosidase libère des sucres fermentables à partir de la matière de fibre de maïs issue d'une matière première principalement de grains de maïs qui sinon ne serait pas disponible pour une fermentation, dans lequel la α-glucosidase est une enzyme GH31 et dans lequel la mise en contact avec la α-glucosidase est mise en œuvre en présence d'une ou plusieurs enzymes supplémentaires.

2. Procédé selon la revendication 1, dans lequel la matière de fibre de maïs est la drêche résultant de la distillation d'un produit de fermentation, ou un dérivé de celle-ci.

3. Procédé selon la revendication 1, dans lequel la matière de fibre de maïs est produite en séparant physiquement la fibre de maïs à partir de l'amidon dans un substrat pour la fermentation d'éthanol.

4. Procédé selon la revendication 1, dans lequel la matière de fibre de maïs est exposée à une température d'au moins 80°C avant la mise en contact avec la α-glucosidase.

5. Procédé selon la revendication 1, dans lequel la matière de fibre de maïs est soumise à une étape de distillation alcoolique avant la mise en contact avec la α-glucosidase.

6. Procédé selon la revendication 1, dans lequel la matière de fibre de maïs contient 1 à 12% de fibre de maïs (p/p).

7. Procédé selon l'une quelconque des revendications précédentes, dans lequel le prétraitement est un prétraitement acide ou un prétraitement alcalin.

8. Procédé selon l'une quelconque des revendications précédentes, dans lequel la α-glucosidase a au moins 70% d'identité de séquence d'acides aminés avec un polypeptide ayant une séquence d'acides aminés choisie dans le groupe constitué par SEQ ID NO: 1, SEQ ID NO: 2, SEQ ID NO: 3 et SEQ ID NO: 4.

9. Procédé pour augmenter la production de sucres fermentables à partir de drêche prétraitée chimiquement, comprenant:

   la fermentation d'une matière première principalement de grains de maïs contenant au moins 80% de grains de maïs avec éthanolagène pour produire de l'éthanol;
   l'évaporation d'au moins une partie de l'éthanol et la récupération de la drêche;
   le prétraitement chimique de la drêche;
   la mise en contact de la drêche avec de la α-glucosidase (EC 3.2.1.20) pour libérer des sucres fermentables,
   la fermentation des sucres fermentables libérés avec un éthanolagène pour produire de l'éthanol supplémentaire; et
   la récupération de l'éthanol supplémentaire;
   dans lequel la mise en contact avec la α-glucosidase libère des sucres fermentables à partir de la matière de fibre de maïs issue de la matière première principalement de grains de maïs qui sinon ne serait pas disponible pour une fermentation,
   dans lequel la α-glucosidase est une enzyme GH31 et dans lequel la mise en contact avec la α-glucosidase est mise en œuvre en présence d'une ou plusieurs enzymes supplémentaires.

10. Procédé selon la revendication 9, dans lequel le prétraitement est un prétraitement acide ou un prétraitement alcalin.

11. Procédé selon l'une quelconque des revendications 9 à 10, dans lequel la matière de fibre de maïs contient 1 à 12% de fibre de maïs (p/p).

12. Procédé selon l'une quelconque des revendications 9 à 11, dans lequel la α-glucosidase a au moins 70% d'identité de séquence d'acides aminés avec un polypeptide ayant une séquence d'acides aminés choisie dans le groupe constitué par SEQ ID NO: 1, SEQ ID NO: 2, SEQ ID NO: 3 et SEQ ID NO: 4.

**13.** Polypeptide ayant une activité de α-glucosidase, choisi dans le groupe constitué par:

(a) un polypeptide comprenant une séquence d'acides aminés ayant au moins 97% d'identité avec le polypeptide de SEQ ID NO: 3;
(b) un polypeptide mature produit par le traitement du polypeptide de SEQ ID NO: 6 par modification par signal peptidase ou post-traductionnelle pendant la sécrétion à partir d'un hôte d'expression.

**14.** Polypeptide selon la revendication 13, dans lequel l'alternative (a) comprend une séquence d'acides aminés ayant au moins 99% d'identité avec le polypeptide de SEQ ID NO: 3.

**15.** Polynucléotide comprenant une séquence de nucléotides qui code le polypeptide selon la revendication 13 ou la revendication 14.

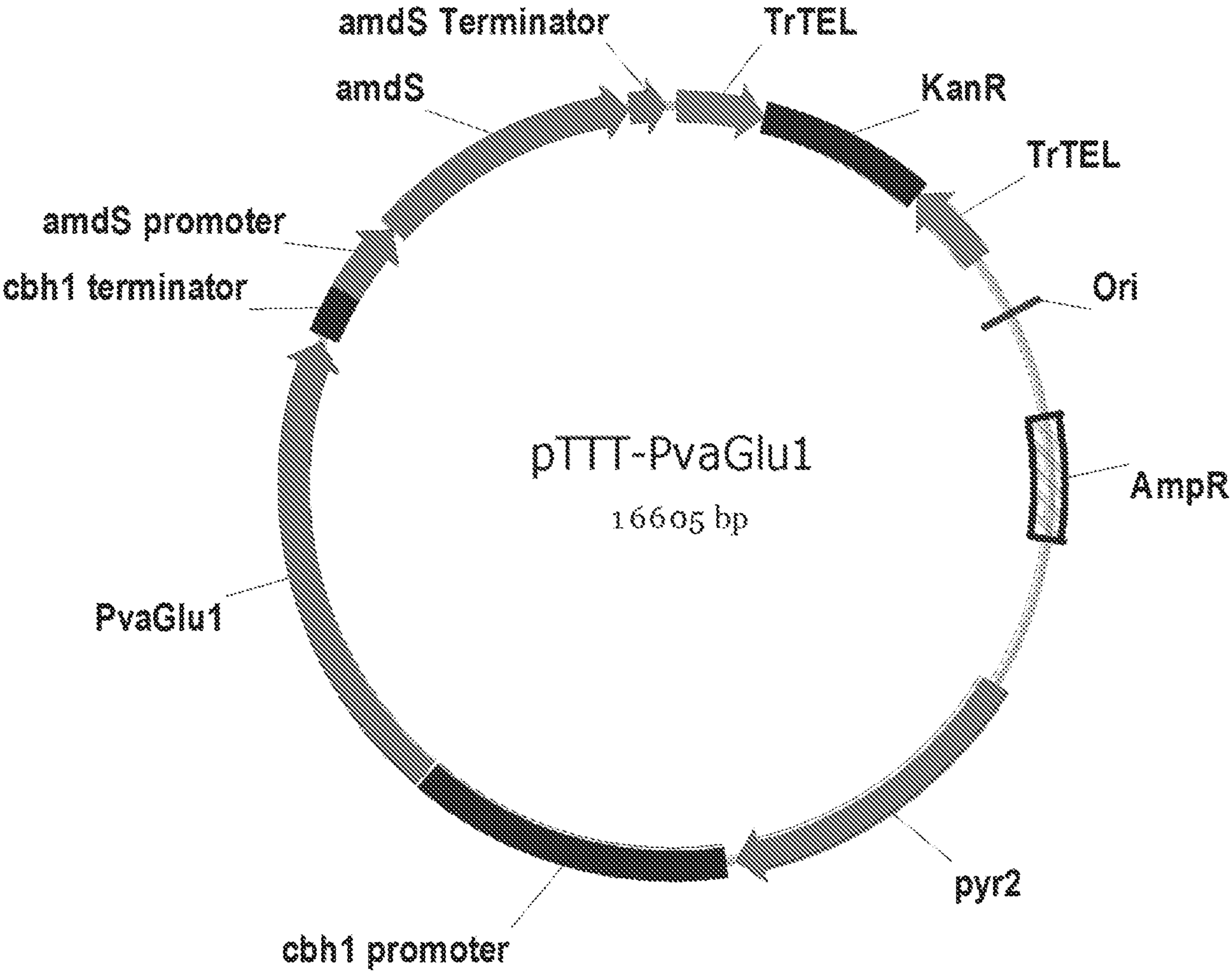
amdS Terminator
TrTEL
amdS
KanR
TrTEL
amdS promoter
cbh1 terminator
Ori
pTTT-PvaGlu1
16605 bp
AmpR
PvaGlu1
pyr2
cbh1 promoter

Figure 1

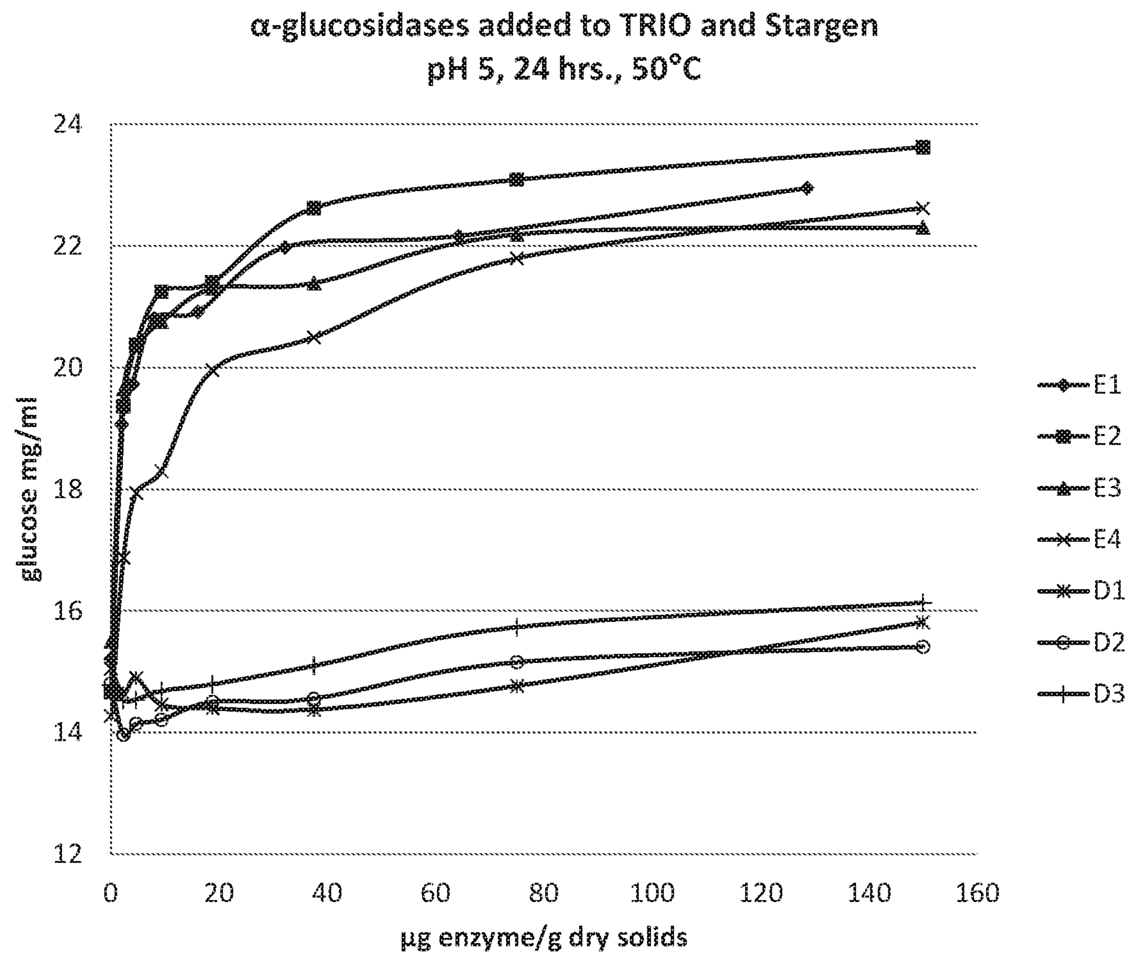
α-glucosidases added to TRIO and Stargen
pH 5, 24 hrs., 50°C
24
22
20
18
16
14
12
glucose mg/ml
0
20
40
60
80
100
120
140
160
µg enzyme/g dry solids
E1
E2
E3
E4
D1
D2
D3

Figure 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2016138315 A
- US 8633033 B
- WO 2015017256 A
- WO 05001036 A
- US 8633003 B
- US 1766737 W
- US 62434883 B

### Non-patent literature cited in the description


- **OKUYAMA, M. et al.** *Cell. Mol. Life Sci.,* 2016, vol. 73, 2727-51
- **TE'O et al.** *J. Microbiol. Methods,* 2002, vol. 51, 393-99
- **NGO, T.T. ; LENHOFF, H.M.** *Anal. Biochem.,* 1980, vol. 105, 389-397

SEQUENCE LISTING

<110> Danisco US Inc.

<120> Method for Increasing the Production of Ethanol from a Product in a Starch Hydrolysis Process

<130> NB41166-WO-PCT

<140> PCT/US17/66737
<141> 2017-12-15

<150> 62/434883
<151> 2016-12-15

<160> 7

<170> PatentIn version 3.5

<210> 1
<211> 960
<212> PRT
<213> Aspergillus niger

<400> 1

Ser Thr Thr Ala Pro Ser Gln Pro Gln Phe Thr Ile Pro Ala Ser Ala
1 5 10 15

Asp Val Gly Ala Gln Leu Ile Ala Asn Ile Asp Asp Pro Gln Ala Ala
20 25 30

Asp Ala Gln Ser Val Cys Pro Gly Tyr Lys Ala Ser Lys Val Gln His
35 40 45

Asn Ser Arg Gly Phe Thr Ala Ser Leu Gln Leu Ala Gly Arg Pro Cys
50 55 60

Asn Val Tyr Gly Thr Asp Val Glu Ser Leu Thr Leu Ser Val Glu Tyr
65 70 75 80

Gln Asp Ser Asp Arg Leu Asn Ile Gln Ile Leu Pro Thr His Val Asp
85 90 95

Ser Thr Asn Ala Ser Trp Tyr Phe Leu Ser Glu Asn Leu Val Pro Arg
100 105 110

Pro Lys Ala Ser Leu Asn Ala Ser Val Ser Gln Ser Asp Leu Phe Val
115 120 125

Ser Trp Ser Asn Glu Pro Ser Phe Asn Phe Lys Val Ile Arg Lys Ala
130 135 140

Thr Gly Asp Ala Leu Phe Ser Thr Glu Gly Thr Val Leu Val Tyr Glu

```
145                 150                 155                 160

Asn Gln Phe Ile Glu Phe Val Thr Ala Leu Pro Glu Glu Tyr Asn Leu
                165                 170                 175

Tyr Gly Leu Gly Glu His Ile Thr Gln Phe Arg Leu Gln Arg Asn Ala
            180                 185                 190

Asn Leu Thr Ile Tyr Pro Ser Asp Asp Gly Thr Pro Ile Asp Gln Asn
        195                 200                 205

Leu Tyr Gly Gln His Pro Phe Tyr Leu Asp Thr Arg Tyr Tyr Lys Gly
    210                 215                 220

Asp Arg Gln Asn Gly Ser Tyr Ile Pro Val Lys Ser Ser Glu Ala Asp
225                 230                 235                 240

Ala Ser Gln Asp Tyr Ile Ser Leu Ser His Gly Val Phe Leu Arg Asn
                245                 250                 255

Ser His Gly Leu Glu Ile Leu Leu Arg Ser Gln Lys Leu Ile Trp Arg
            260                 265                 270

Thr Leu Gly Gly Gly Ile Asp Leu Thr Phe Tyr Ser Gly Pro Ala Pro
        275                 280                 285

Ala Asp Val Thr Arg Gln Tyr Leu Thr Ser Thr Val Gly Leu Pro Ala
    290                 295                 300

Met Gln Gln Tyr Asn Thr Leu Gly Phe His Gln Cys Arg Trp Gly Tyr
305                 310                 315                 320

Asn Asn Trp Ser Asp Leu Ala Asp Val Val Ala Asn Phe Glu Lys Phe
                325                 330                 335

Glu Ile Pro Leu Glu Tyr Ile Trp Thr Asp Ile Asp Tyr Met His Gly
            340                 345                 350

Tyr Arg Asn Phe Asp Asn Asp Gln His Arg Phe Ser Tyr Ser Glu Gly
        355                 360                 365

Asp Glu Phe Leu Ser Lys Leu His Glu Ser Gly Arg Tyr Tyr Val Pro
    370                 375                 380

Ile Val Asp Ala Ala Leu Tyr Ile Pro Asn Pro Glu Asn Ala Ser Asp
385                 390                 395                 400
```

Ala Tyr Ala Thr Tyr Asp Arg Gly Ala Ala Asp Asp Val Phe Leu Lys
405 410 415

Asn Pro Asp Gly Ser Leu Tyr Ile Gly Ala Val Trp Pro Gly Tyr Thr
420 425 430

Val Phe Pro Asp Trp His His Pro Lys Ala Val Asp Phe Trp Ala Asn
435 440 445

Glu Leu Val Ile Trp Ser Lys Lys Val Ala Phe Asp Gly Val Trp Tyr
450 455 460

Asp Met Ser Glu Val Ser Ser Phe Cys Val Gly Ser Cys Gly Thr Gly
465 470 475 480

Asn Leu Thr Leu Asn Pro Ala His Pro Ser Phe Leu Leu Pro Gly Glu
485 490 495

Pro Gly Asp Ile Ile Tyr Asp Tyr Pro Glu Ala Phe Asn Ile Thr Asn
500 505 510

Ala Thr Glu Ala Ala Ser Ala Ser Ala Gly Ala Ser Ser Gln Ala Ala
515 520 525

Ala Thr Ala Thr Thr Thr Ser Thr Ser Val Ser Tyr Leu Arg Thr Thr
530 535 540

Pro Thr Pro Gly Val Arg Asn Val Glu His Pro Pro Tyr Val Ile Asn
545 550 555 560

His Asp Gln Glu Gly His Asp Leu Ser Val His Ala Val Ser Pro Asn
565 570 575

Ala Thr His Val Asp Gly Val Glu Glu Tyr Asp Val His Gly Leu Tyr
580 585 590

Gly His Gln Gly Leu Asn Ala Thr Tyr Gln Gly Leu Leu Glu Val Trp
595 600 605

Ser His Lys Arg Arg Pro Phe Ile Ile Gly Arg Ser Thr Phe Ala Gly
610 615 620

Ser Gly Lys Trp Ala Gly His Trp Gly Gly Asp Asn Tyr Ser Lys Trp
625 630 635 640

Trp Ser Met Tyr Tyr Ser Ile Ser Gln Ala Leu Ser Phe Ser Leu Phe
645 650 655

Gly Ile Pro Met Phe Gly Ala Asp Thr Cys Gly Phe Asn Gly Asn Ser
660 665 670

Asp Glu Glu Leu Cys Asn Arg Trp Met Gln Leu Ser Ala Phe Phe Pro
675 680 685

Phe Tyr Arg Asn His Asn Glu Leu Ser Thr Ile Pro Gln Glu Pro Tyr
690 695 700

Arg Trp Ala Ser Val Ile Glu Ala Thr Lys Ser Ala Met Arg Ile Arg
705 710 715 720

Tyr Ala Ile Leu Pro Tyr Phe Tyr Thr Leu Phe Asp Leu Ala His Thr
725 730 735

Thr Gly Ser Thr Val Met Arg Ala Leu Ser Trp Glu Phe Pro Asn Asp
740 745 750

Pro Thr Leu Ala Ala Val Glu Thr Gln Phe Met Val Gly Pro Ala Ile
755 760 765

Met Val Val Pro Val Leu Glu Pro Leu Val Asn Thr Val Lys Gly Val
770 775 780

Phe Pro Gly Val Gly His Gly Glu Val Trp Tyr Asp Trp Tyr Thr Gln
785 790 795 800

Ala Ala Val Asp Ala Lys Pro Gly Val Asn Thr Thr Ile Ser Ala Pro
805 810 815

Leu Gly His Ile Pro Val Tyr Val Arg Gly Gly Asn Ile Leu Pro Met
820 825 830

Gln Glu Pro Ala Leu Thr Thr Arg Glu Ala Arg Gln Thr Pro Trp Ala
835 840 845

Leu Leu Ala Ala Leu Gly Ser Asn Gly Thr Ala Ser Gly Gln Leu Tyr
850 855 860

Leu Asp Asp Gly Glu Ser Ile Tyr Pro Asn Ala Thr Leu His Val Asp
865 870 875 880

Phe Thr Ala Ser Arg Ser Ser Leu Arg Ser Ser Ala Gln Gly Arg Trp
885 890 895

Lys Glu Arg Asn Pro Leu Ala Asn Val Thr Val Leu Gly Val Asn Lys
900 905 910

Glu Pro Ser Ala Val Thr Leu Asn Gly Gln Ala Val Phe Pro Gly Ser
915 920 925

Val Thr Tyr Asn Ser Thr Ser Gln Val Leu Phe Val Gly Gly Leu Gln
930 935 940

Asn Leu Thr Lys Gly Gly Ala Trp Ala Glu Asn Trp Val Leu Glu Trp
945 950 955 960

<210> 2
<211> 985
<212> PRT
<213> Rasamsonia composticola

<400> 2

Ala Ala Ile Val Arg Arg Asn Gly Ala Ser Pro Ser Cys Pro Gly Tyr
1 5 10 15

Lys Ala Ser Asn Val Lys Thr Val Asp Gly Glu Ile Val Ser Ala Asp
20 25 30

Leu Asn Leu Ala Gly Pro Ala Cys Asn Val Tyr Gly Thr Asp Leu Asp
35 40 45

Asp Leu Lys Leu Gln Val Glu Tyr Gln Ser Glu Gln Arg Leu His Val
50 55 60

Lys Ile Tyr Asp Ala Ala Glu Gln Val Tyr Gln Val Pro Thr Ala Val
65 70 75 80

Leu Pro Arg Pro Ser Ser Ala Asn Ile Pro Pro Ala Lys Ser Asp Leu
85 90 95

Lys Phe Ser Met Thr Asn Asp Pro Phe Ser Phe Thr Ile Lys Arg Arg
100 105 110

Ser Asn Gly Glu Ile Leu Phe Asp Thr Ser Gly His Pro Leu Ile Phe
115 120 125

Glu Ser Gln Tyr Leu Gly Leu Arg Thr Lys Leu Pro Asp Ser Pro Asn
130 135 140

Ile Tyr Gly Leu Gly Glu His Thr Gly Ser Phe Arg Leu Pro Thr Lys
145 150 155 160

Asn Tyr Thr Arg Thr Leu Trp Ser Arg Asp Ala Tyr Gly Thr Pro Lys
165 170 175

```
Asp Thr Asn Leu Tyr Gly Asn His Pro Val Tyr Phe Asp Tyr Arg Gly
            180                 185                 190

Ser Asn Gly Thr His Gly Val Phe Leu Leu Asn Ser Asn Gly Met Asp
        195                 200                 205

Val Asp Ile Asp Val Asp Ser Asp Gly Gln Tyr Leu Gln Tyr Asn Thr
    210                 215                 220

Leu Gly Gly Val Leu Asp Phe Tyr Phe Leu Ser Gly Pro Asp Pro Lys
225                 230                 235                 240

Ala Val Ala Thr Gln Tyr Ala Glu Thr Val Gly Lys Pro Val Met Met
                245                 250                 255

Pro Tyr Trp Gly Phe Gly Phe His Asn Cys Arg Tyr Gly Tyr Gln Asp
            260                 265                 270

Ile Tyr Glu Val Ala Glu Ile Ile Ala Asn Tyr Ser Ala Ala Asn Ile
        275                 280                 285

Pro Leu Glu Thr Gln Trp Thr Asp Ile Asp Tyr Met Asp Leu Arg Lys
    290                 295                 300

Val Phe Thr Leu Asp Pro Tyr Arg Tyr Pro Leu Lys Leu Val Gln Glu
305                 310                 315                 320

Val Val Ser Tyr Leu His Lys His Asn Gln His Tyr Ile Met Met Val
                325                 330                 335

Asp Pro Ala Val Ala Tyr Gln Asn Tyr Ser Ala Phe Asn Asn Gly Val
            340                 345                 350

Ala Ala Asp Ala Phe Leu Lys Phe Ser Asn Gly Ser Ile Tyr Gln Gly
        355                 360                 365

Val Val Trp Pro Gly Pro Thr Ala Phe Pro Asp Trp Phe Ala Pro Gln
    370                 375                 380

Thr Gln Glu Phe Trp Asn Ser Glu Phe Ser Thr Phe Phe Asp Pro Ala
385                 390                 395                 400

His Gly Val Asp Ile Asp Ala Leu Trp Ile Asp Met Asn Glu Ala Ser
                405                 410                 415

Asn Phe Cys Asp Phe Pro Cys Ser Asn Pro Ala Ala Tyr Ala Ala Ala
```

420 425 430

Asn Gly Asp Pro Pro Thr Pro Pro Pro Val Arg Leu Ser Pro Pro Arg
435 440 445

Pro Ile Pro Gly Phe Gly Pro Asp Phe Gln Pro Thr Cys Val Ala Thr
450 455 460

Val Ser Phe Asp Cys Asp Ala Gln Thr Tyr Phe Gly Glu Asn Ile Leu
465 470 475 480

Ile Leu Gly Asn Ser Thr Thr Leu Gly Ala Gly Asp Val His Met Ala
485 490 495

Pro Val Met Ser Ala Asn Asn Tyr Pro Ile Trp Gln Leu Thr Val Gln
500 505 510

Met Pro Pro Asn Gly Thr Phe Ser Tyr Gln Tyr Val Arg Lys Glu Ser
515 520 525

Asp Gly Ser Tyr Ile Tyr Glu Gln Thr Asn Arg Thr Val Thr Thr Gly
530 535 540

Asp Cys Thr Ser Gly Thr Leu Lys Val Ser Asp Thr Ile Thr Thr Ser
545 550 555 560

Ser Gly Pro His Lys Arg Ser Glu Leu Arg Pro Leu Val Arg Ser Pro
565 570 575

Phe Pro Ala Glu Asp Leu Thr Arg Arg Gln Ser Gly Ser Met Leu Gly
580 585 590

Leu Pro Asn Arg Asn Leu Leu Asn Pro Pro Tyr Thr Ile His Asn Ala
595 600 605

Ala Gly Asn Leu Ser Glu Lys Thr Ile Asn Thr Asp Leu Ile His Ala
610 615 620

Gly Gly Tyr Ala Glu Tyr Asp Thr His Asn Leu Tyr Gly Thr Met Met
625 630 635 640

Ser Ala Thr Ser Arg Glu Ala Met Leu Asn Arg Arg Pro Ala Val Arg
645 650 655

Pro Leu Val Ile Thr Arg Ser Thr Phe Ala Gly Ala Gly Arg Gln Val
660 665 670

```
Gly His Trp Leu Gly Asp Asn Phe Ala Asp Trp Asp His Tyr Arg Trp
        675                 680                 685

Thr Ile Ala Glu Leu Gln Glu Phe Ala Ala Leu Phe Gln Ile Pro Met
    690                 695                 700

Val Gly Ser Asp Ile Cys Gly Tyr Asp Gly Asn Thr Thr Asp Asn Leu
705                 710                 715                 720

Cys Ser Arg Trp Val Phe Leu Gly Ala Phe Ser Pro Phe Phe Arg Asp
                725                 730                 735

His Ser Asp Asn Gln Ser Pro Pro His Glu Leu Tyr Arg Thr Pro Gln
            740                 745                 750

Ile Ala Ala Ala Ala Arg Ala Ala Ile Asp Ile Arg Tyr Arg Leu Leu
        755                 760                 765

Asp Tyr Ala Tyr Thr Val Leu Trp Thr Gln Thr Gln Thr Gly Ala Pro
    770                 775                 780

Met Leu Asn Pro Met Phe Phe Glu Tyr Pro Ala Asp Ser Asn Thr Ala
785                 790                 795                 800

Asp Leu Gln Tyr Gln Phe Phe Trp Gly Asp Ser Ile Met Val Ala Pro
                805                 810                 815

Val Thr Asp Asn Asp Ser Thr Thr Val Asn Val Tyr Phe Pro Lys Asp
            820                 825                 830

Gln Phe Tyr Asp Phe Tyr Thr Gly Ala Pro Val Ser Gly Glu Gly Asn
        835                 840                 845

Thr Val Thr Leu Thr Asp Val Gly Phe Asp Thr Ile Pro Leu Tyr Phe
    850                 855                 860

Lys Gly Gly Ser Ile Val Pro Met Arg Val Arg Ser Ala Asn Thr Thr
865                 870                 875                 880

Ala Glu Leu Arg Gln Gln Asp Phe Val Val Val Ile Ala Pro Asp Ser
                885                 890                 895

His Gly Asp Ala Thr Gly Gln Leu Tyr Leu Asp Asp Gly Glu Ser Ile
            900                 905                 910

Asn Gln Pro His Thr Ser Glu Ile Gln Phe Ser Tyr Arg Gly Gly His
        915                 920                 925
```

Phe Ser Met Thr Gly Lys Phe Asp Tyr Asp Pro Gly Asn Val Val Ile
    930                 935                 940

Ser Gln Ile Thr Leu Leu Gly Ala Asp Gly Ala Gly Lys Gly Gly Ser
945                 950                 955                 960

Tyr Asn Ser Thr Thr Lys Val Ala Thr Tyr Lys Val Asn Ala Lys Leu
                965                 970                 975

Thr Gly Lys Phe Glu Ala Ser Leu His
            980                 985

<210> 3
<211> 968
<212> PRT
<213> Paecilomyces variotii

<400> 3

Ala Ala Ile Ser Pro Ala Ala Thr Ser Ser Thr Ala Ser Trp Gly Pro
1               5                   10                  15

Val Phe Thr Val Pro Ala Ser Ala Asp Glu Gly Ala Gln Leu Ile Ala
            20                  25                  30

Asn Ile Asn Asp Pro Gln Ser Val Asn Ala Gln Thr Val Cys Pro Gly
        35                  40                  45

Tyr Val Ala Ser Asn Val Gln Asn Asn Glu Phe Gly Phe Thr Ala Thr
    50                  55                  60

Leu Asn Leu Ala Gly Lys Ala Cys Asn Val Tyr Gly Thr Asp Val Asp
65                  70                  75                  80

Ser Leu Asn Leu Thr Val Gln Tyr Gln Ala Ser Asp Arg Leu Asn Ile
                85                  90                  95

Asn Ile Gly Pro Ala His Val Asp Ala Ser Asn Glu Ser Trp Tyr Ile
            100                 105                 110

Leu Ser Asp Asp Leu Val Tyr Lys Pro Thr Val Asp Gly Thr Ala Ser
        115                 120                 125

Ile Ser Gln Ser Asp Leu Leu Val Ser Trp Ser Asn Glu Pro Ser Phe
    130                 135                 140

Asn Phe Lys Val Ile Arg Lys Ala Asn Lys Asp Val Leu Phe Asn Thr
145                 150                 155                 160

```
Glu Gly Thr Val Leu Val Tyr Glu Asn Gln Phe Ile Glu Phe Val Ser
                165                 170                 175

Ala Leu Pro Glu Asn Tyr Asn Leu Tyr Gly Leu Gly Glu Arg Ile His
            180                 185                 190

Gly Leu Arg Leu Gly Asn Asn Phe Thr Ala Thr Thr Tyr Ala Ala Asp
        195                 200                 205

Ala Ala Asp Pro Ile Asp Ala Asn Ile Tyr Gly Thr His Pro Phe Tyr
    210                 215                 220

Leu Asp Thr Arg Tyr Tyr Glu Val Asp Ser Lys Gln Gly Thr Tyr Thr
225                 230                 235                 240

Leu Leu Thr Thr Asn Glu Thr Asp Gln Ser Lys Glu Tyr Thr Ser Phe
                245                 250                 255

Ser His Gly Val Phe Leu Arg Asn Ala His Gly Gln Glu Val Leu Leu
            260                 265                 270

Arg Pro Glu Gly Ile Thr Trp Arg Thr Leu Gly Gly Ser Ile Asp Leu
        275                 280                 285

Tyr Phe Tyr Ser Gly Pro Thr Gln Ala Asp Val Thr Arg Ser Tyr Gln
    290                 295                 300

Thr Ser Thr Val Gly Leu Pro Thr Met Gln Gln Tyr Tyr Thr Phe Gly
305                 310                 315                 320

Tyr His Gln Cys Arg Trp Gly Tyr Gln Asn Trp Ser Val Met Ala Asp
                325                 330                 335

Val Val Ser Ser Phe Ala Lys Phe Gln Ile Pro Leu Glu Thr Ile Trp
            340                 345                 350

Ser Asp Ile Asp Tyr Met Asn Ala Tyr Arg Asp Phe Glu Asn Asp Pro
        355                 360                 365

Ile Arg Phe Ser Tyr Ser Glu Gly Ala Glu Phe Leu Gly Gln Leu His
    370                 375                 380

Glu Asn Gly Gln His Trp Val Pro Ile Val Asp Ser Ala Ile Tyr Ile
385                 390                 395                 400

Pro Asn Ser Glu Asn Ala Ser Asp Ala Tyr Asp Val Tyr Thr Arg Gly
                405                 410                 415
```

```
Glu Ala Asp Gly Val Trp Met Thr Asn Pro Asp Gly Ser Leu Tyr Ile
            420                 425                 430

Gly Ala Val Trp Pro Gly Tyr Thr Val Phe Pro Asp Trp His Asn Pro
        435                 440                 445

Lys Ala His Glu Phe Trp Ser Asn Glu Ile Ala Thr Trp His Gln Lys
    450                 455                 460

Val Ala Phe Asp Gly Ile Trp Ile Asp Met Ser Glu Val Ser Ser Phe
465                 470                 475                 480

Cys Val Gly Ser Cys Gly Thr Gly Asn Leu Thr Leu Asn Pro Val His
                485                 490                 495

Pro Ser Phe Leu Leu Pro Gly Glu Pro Gly Ala Val Ile Tyr Asp Tyr
            500                 505                 510

Pro Glu Ser Phe Asn Val Thr Asn Ser Thr Glu Ala Ala Ser Ala Ser
        515                 520                 525

Ala Ala Ser Val Ser Gln Ala Ala Ala Thr Ala Ser Ala Ser Ala Ser
    530                 535                 540

Thr Thr Thr Ser Tyr Leu Arg Thr Thr Pro Thr Pro Gly Val Arg Asp
545                 550                 555                 560

Val Asn His Pro Pro Tyr Val Ile Asn Asn Val Gln Pro Gly His Asp
                565                 570                 575

Leu Ala Val His Ala Val Ser Pro Asn Ala Thr His Ile Asp Gly Val
            580                 585                 590

Ser Glu Tyr Asp Val His Asn Leu Trp Gly Tyr Gln Ile Leu Asn Ala
        595                 600                 605

Thr Tyr His Gly Leu Leu Lys Val Trp Glu Asp Lys Arg Pro Phe Ile
    610                 615                 620

Ile Gly Arg Ser Thr Phe Ala Gly Ser Gly Lys Trp Ala Gly His Trp
625                 630                 635                 640

Gly Gly Asp Asn Thr Ser Leu Trp Ala Tyr Met Phe Phe Ser Ile Pro
                645                 650                 655

Gln Ala Leu Ser Phe Ser Leu Phe Gly Ile Pro Met Phe Gly Val Asp
```

```
            660                 665                 670

Thr Cys Gly Phe Asn Gly Asn Ser Asp Glu Glu Leu Cys Asn Arg Trp
        675                 680                 685

Met Gln Leu Ser Ala Phe Phe Pro Phe Tyr Arg Asn His Asn Val Leu
    690                 695                 700

Ser Ala Ile Ser Gln Glu Pro Tyr Val Trp Ala Ser Val Ile Asp Ala
705                 710                 715                 720

Ser Lys Ala Ala Met Lys Ile Arg Tyr Ala Leu Leu Pro Tyr Ile Tyr
                725                 730                 735

Thr Leu Phe Tyr Leu Ala His Thr Thr Gly Ser Thr Val Met Arg Ala
            740                 745                 750

Val Ser Trp Glu Phe Pro Asn Asp Pro Ser Leu Ala Ala Ile Asp Thr
        755                 760                 765

Gln Phe Leu Leu Gly Pro Ser Leu Met Val Val Pro Val Leu Glu Pro
    770                 775                 780

Gln Val Asp Tyr Val Lys Gly Val Phe Pro Gly Val Gly Asn Gly Glu
785                 790                 795                 800

Val Trp Tyr Asp Trp Tyr Thr Gln Ser Val Phe Asp Ala Lys Pro Gly
                805                 810                 815

Val Asn Thr Thr Ile Ser Ala Pro Leu Gly His Ile Pro Val Phe Val
            820                 825                 830

Arg Gly Gly Ser Ile Leu Pro Met Gln Glu Pro Ala Leu Thr Thr Arg
        835                 840                 845

Asp Ala Arg Lys Thr Pro Trp Ala Leu Leu Thr Ala Leu Gly Gly Asn
    850                 855                 860

Gly Thr Ala Ser Gly Gln Leu Tyr Ile Asp Asp Gly Glu Ser Ile Thr
865                 870                 875                 880

Pro Asn Ala Thr Leu Asn Val Asp Phe Val Ala Ser Asn Ser Asn Leu
                885                 890                 895

Val Ala Ser Pro Arg Gly Ser Trp Val Glu Lys Asn Pro Leu Ala Asn
            900                 905                 910
```

```
Val Thr Val Leu Gly Val Pro Thr Ala Pro Ser Ser Val Thr Phe Asn
        915                 920                 925

Gly Ala Ala Val Pro Arg Ala Ser Val Ala Tyr Asn Ser Thr Ser Lys
    930                 935                 940

Thr Leu Phe Val Gly Gly Leu Gln Asp Phe Thr Lys Thr Gly Ala Trp
945                 950                 955                 960

Ala Asp Lys Trp Val Leu Lys Trp
                965
```

<210> 4
<211> 965
<212> PRT
<213> Penicillium russelli

<400> 4

```
Ala Ala Thr Gln Thr Ser Ser Ser Ala Tyr Val Gln Thr Thr Leu Glu
1               5                   10                  15

Ser Ser Val Asp Val Gly Ala Asn Leu Ile Ala Asn Ile Asp Asp Pro
            20                  25                  30

Glu Ala Ile Asn Ala Gln Ser Ala Cys Pro Gly Tyr Arg Ala Ser Asn
        35                  40                  45

Val Gln Asn Thr Ser Arg Gly Trp Thr Ala Thr Leu Lys Leu Ala Gly
    50                  55                  60

Lys Ala Cys Asn Val Tyr Gly Thr Asp Val Glu Ser Leu Asn Phe Thr
65                  70                  75                  80

Leu Glu Tyr Leu Ser Ser Thr Arg Val Asn Ile Gln Ile Thr Pro Ser
                85                  90                  95

His Val Asp Ser Ser Asn Ala Ser Trp Tyr His Leu Ser Glu Asp Val
            100                 105                 110

Val Pro Arg Pro Lys Ala Asp Lys Asn Ala Ser Ala Lys Asp Ser His
        115                 120                 125

Phe Glu Val Ser Trp Ser Asn Glu Pro Ser Phe Gly Phe Lys Val Ala
    130                 135                 140

Arg Lys Ala Thr Gly Asp Val Leu Phe Asn Thr Ile Gly Ser Lys Leu
145                 150                 155                 160
```

```
Val Tyr Glu Asn Gln Phe Ile Glu Phe Val Thr Ala Leu Pro Asp Asp
                165                 170                 175

Tyr Asn Leu Tyr Gly Leu Gly Glu His Ile Gln Gln Leu Arg Leu Leu
            180                 185                 190

Lys Asn Ser Thr Phe Thr Leu Tyr Ala Ala Asp Thr Gly Asp Pro Val
        195                 200                 205

Asp Leu Asn Thr Tyr Gly Ser His Ala Phe Tyr Leu Asp Thr Arg Tyr
    210                 215                 220

Tyr Glu Val Asn Asp Lys Gly Ser His Thr Leu Val Ser Ser Asp Gln
225                 230                 235                 240

Ala Thr Thr Ser Lys Asn Tyr Val Ser Tyr Ser His Gly Val Phe Leu
                245                 250                 255

Arg Asn Ala His Gly Gln Glu Ile Leu Leu Gly Thr Gly Lys Leu Thr
            260                 265                 270

Trp Arg Thr Ile Gly Gly Ser Ile Asp Leu Thr Leu Tyr Ala Gly Pro
        275                 280                 285

Thr Gln Thr Glu Val Thr Lys Asp Tyr Gln Leu Ser Thr Ile Gly Leu
    290                 295                 300

Pro Ala Met Gln Gln Tyr Phe Thr Phe Gly Tyr His Gln Cys Arg Trp
305                 310                 315                 320

Gly Tyr Thr Asn Trp Ser Glu Val Glu Asp Val Val Ala Asn Phe Gln
                325                 330                 335

Lys Phe Glu Ile Pro Leu Glu Asn Ile Trp Asn Asp Ile Asp Tyr Met
            340                 345                 350

His Gly Tyr Arg Asp Phe Asp Asn Asp Gln Asn Arg Tyr Ser Tyr Glu
        355                 360                 365

Glu Gly Ala Val Phe Leu Glu Lys Leu His Lys Ala Gly Ile His Tyr
    370                 375                 380

Ile Pro Ile Val Asp Ser Ala Leu Tyr Ile Pro Asp Pro Asn Asn Ala
385                 390                 395                 400

Ser Asp Ala Tyr Asp Thr Tyr Thr Arg Gly Ala Glu Leu Asp Val Phe
                405                 410                 415
```

Leu Lys Asn Pro Asp Gly Ser Thr Tyr Ile Gly Ala Val Trp Pro Gly
420 425 430

Tyr Thr Val Phe Ala Asp Trp His His Pro Lys Ala Gly Asp Phe Trp
435 440 445

Ala Asn Glu Leu Val Thr Trp His Glu Arg Val Ala Phe Asp Gly Ile
450 455 460

Trp Ile Asp Met Asn Glu Val Ser Ser Phe Cys Val Gly Ser Cys Gly
465 470 475 480

Ser Gly Lys Leu Ser Gln Asn Pro Val His Pro Pro Phe Ser Leu Pro
485 490 495

Gly Glu Pro Gly Asn Ile Ile Tyr Asp Tyr Pro Glu Gly Phe Asn Ala
500 505 510

Thr Asn Ser Thr Glu Ala Ala Ser Ala Ser Ala Ala Ser Ala Ser Gln
515 520 525

Ala Ser Ala Ala Ala Ala Thr Gly Gln Ser Ala Ala Thr Thr Thr Thr
530 535 540

Pro Tyr Leu Arg Thr Thr Pro Thr Pro Gly Val Arg Asp Val Asn His
545 550 555 560

Pro Pro Tyr Val Ile Asn His Ala Gln Thr Gly His Asp Leu Ala Val
565 570 575

His Ala Val Ser Pro Asn Ala Thr His Ser Asp Gly Val Gln Glu Tyr
580 585 590

Asp Val His Ser Leu Tyr Gly His Ser Ile Ile Arg Ala Thr Tyr Glu
595 600 605

Gly Leu Leu Lys Val Phe Pro Glu Lys Arg Pro Phe Ile Ile Gly Arg
610 615 620

Ser Thr Phe Ala Gly Thr Gly Lys Trp Ala Gly His Trp Gly Gly Asp
625 630 635 640

Asn Asn Ser Lys Trp Ser Tyr Met Phe Trp Ser Ile Pro Gln Ala Leu
645 650 655

Gln Phe Ser Leu Phe Gly Val Pro Met Phe Gly Val Asp Thr Cys Gly
660 665 670

```
Phe Asn Gly Asn Thr Asp Glu Glu Leu Cys Asn Arg Trp Met Gln Leu
        675                 680                 685

Ser Ala Phe Phe Pro Phe Tyr Arg Asn His Asn Val Leu Ser Ala Ile
    690                 695                 700

Ser Gln Glu Pro Tyr Arg Trp Ala Ser Val Ala Glu Ala Ser Lys Ala
705                 710                 715                 720

Ala Met Lys Ile Arg Tyr Ala Ile Leu Pro Tyr Met Tyr Thr Leu Phe
                725                 730                 735

Gln Gln Ala His Thr Thr Gly Ser Thr Val Met Arg Ala Leu Ala Trp
            740                 745                 750

Glu Phe Pro Asn Asp Pro Ser Leu Ala Ala Val Asp Thr Gln Phe Leu
        755                 760                 765

Leu Gly Pro Ser Ile Met Val Val Pro Val Leu Ala Pro Gln Ala Thr
    770                 775                 780

Ser Val Lys Gly Val Phe Pro Gly Ile Lys Gln Gly Glu Val Trp Tyr
785                 790                 795                 800

Asp Trp Tyr Thr Gln Thr Ala Val Asp Ala Gln Pro His Val Asn Thr
                805                 810                 815

Thr Ile Ala Ala Pro Leu Gly His Ile Pro Val Phe Val Arg Gly Asp
            820                 825                 830

Ser Val Leu Pro Met Gln Glu Pro Ala Leu Thr Thr Arg Asp Ala Arg
        835                 840                 845

Asn Thr Pro Trp Thr Ile Leu Ala Ala Leu Gly Asp Lys Gly Thr Ala
    850                 855                 860

Ser Gly Glu Leu Tyr Leu Asp Asp Gly Glu Ser Leu Glu Pro Asn Ala
865                 870                 875                 880

Thr Leu Thr Val Thr Phe Lys Ala Thr Lys Ser Ser Leu Ser Ala Glu
                885                 890                 895

Pro Arg Gly Asn Trp Gln Glu Lys Asn Ala Leu Gly Asn Val Asn Val
            900                 905                 910

Leu Gly Val Ala His Lys Pro Asn Gly Val Thr Leu Asn Gly Lys Ala
```

```
            915                 920                 925
Val Pro Ala Ala Ser Val His Tyr Asn Ser Thr Ser Gln Val Leu Ser
    930                 935                 940
Val Thr Asp Leu Gln Lys Met Thr Ser Lys Gly Ala Phe Ala Asn Asn
945                 950                 955                 960
Trp Val Leu Lys Trp
                965
```

<210> 5
<211> 2961
<212> DNA
<213> Paecilomyces variotii

<400> 5

```
atgggcggct tcacccacta catgctcgct tccgcttggc tgcctctgac cctgggcgcc      60
gccatctctc ccgctgctac cagcagcacc gcttcgtggg gccccgtttt caccgtcccc     120
gcctccgccg acgagggcgc tcagctcatt gccaacatta acgacccccca gtctgttaac    180
gcccagaccg tttgccccgg ctacgttgct tctaacgtcc agaacaacga gttcggcttc     240
accgccaccc tgaacctcgc cggcaaggct tgcaacgttt acggcaccga cgtcgatagc     300
ctgaacctga ccgtccagta ccaggcgagt gaccgactga acattaacat tggccccgcc     360
cacgttgacg cctccaacga gtcttggtac attctgagcg acgacctggt ttacaagcct     420
accgtggacg gcaccgcttc tatctctcag tctgacctgc tggttagctg gtctaacgag     480
ccttctttca acttcaaggt tattcgtaag gctaacaagg acgtcctgtt caacaccgag     540
ggcaccgtcc tcgtttacga gaaccagttc atcgagttcg tcagcgctct ccctgagaac     600
tacaacctgt acggcctggg cgagcgcatt cacggcctgc gactcggcaa caacttcacc     660
gccaccacct acgccgccga cgccgccgac cccatcgacg ctaacatata tggcacccac     720
cctttctacc tggacacccg atactacgag gttgactcca agcagggcac ctacaccctg     780
ctgaccacca acgagaccga ccagtctaag gaatacacct ctttctctca cggcgttttc     840
ctccgaaacg ctcacggcca ggaagttctg ctgcgccctg agggcatcac ctggcgaacc     900
ctgggcggca gcattgacct gtacttctac tccggcccta cccaggctga cgtcacccga     960
tcttaccaga ccagcaccgt tggcctgcct accatgcagc agtactacac cttcggctac    1020
caccagtgcc gatggggcta ccagaactgg tctgtcatgg ctgacgttgt tagctctttc    1080
gccaagttcc agattcctct ggagaccatt tggagcgaca tcgactacat gaacgcttac    1140
cgagacttcg agaacgaccc tatccgattc tcttactctg agggcgctga gttcctcggc    1200
cagctgcacg agaacggcca gcactgggtt cctattgttg actccgccat ctacatccct    1260
```

aacagcgaga acgccagcga cgcttacgac gtttacaccc gaggcgaggc cgacggcgtt 1320
tggatgacca accccgacgg cagcctgtac attggcgccg tttggcccgg ctacaccgtt 1380
ttccccgact ggcacaaccc taaggctcac gagttctggt ctaacgagat tgctacctgg 1440
caccagaagg tcgctttcga cggcatttgg attgacatgt ctgaggtcag ctctttctgc 1500
gttggctctt gcggcaccgg caacctgacc ctcaaccccg tccacccttc tttcctgctc 1560
cccggcgagc ccggcgctgt tatatatgac taccctgagt ctttcaacgt caccaactct 1620
accgaggctg cttctgcttc agccgcatcg gtctcccagg ctgctgctac cgcttctgcc 1680
tctgcctcaa ccaccacctc ttacctgcga accaccccta cccccggcgt ccgagacgtt 1740
aaccaccctc cttacgttat taacaacgtc cagcctggcc acgacctcgc cgtccacgcc 1800
gttagcccca acgctaccca cattgacggc gttagcgagt acgacgtcca caacctgtgg 1860
ggctaccaga ttctgaacgc tacctaccac ggcctcctca aggtttggga ggacaagcgc 1920
cctttcatta ttggccgaag caccttcgct ggcagcggca agtgggctgg ccactggggc 1980
ggcgacaaca cctctctgtg ggcttacatg ttcttcagca ttcctcaggc tctgtctttc 2040
agcctgttcg gcatccccat gttcggcgtt gacacctgcg gcttcaacgg caactccgac 2100
gaagaactgt gcaaccgatg gatgcagctg tctgctttct tccctttcta ccgaaaccac 2160
aacgtcctca gcgctattag ccaagaacct tacgtttggg cctccgtcat tgacgcttct 2220
aaggccgcca tgaagattcg atacgctctg ctgccttaca tatacaccct gttctacctc 2280
gctcacacca ccggcagcac cgtcatgcga gctgtctctt gggagttccc taacgaccct 2340
agcctcgccg ccattgacac ccagttcctc ctgggcccta gcctcatggt tgtccccgtc 2400
ctggagcctc aggttgacta cgttaagggc gttttccccg gcgttggcaa cggcgaggtt 2460
tggtacgact ggtacaccca gtctgttttc gacgccaagc ccggcgttaa caccaccatc 2520
agcgctcctc tcggccacat ccccgttttc gtccgaggcg gcagcattct gcctatgcaa 2580
gagcccgctc tgaccacccg cgacgctcga aagacccctt gggctctcct gaccgctctg 2640
ggcggcaacg gcaccgcttc tggccagctg tacatcgacg acggcgagtc tattacccct 2700
aacgccaccc tgaacgtgga cttcgttgct tccaactcta acctcgttgc gtcacctcgg 2760
ggctcttggg ttgagaagaa ccctctcgct aacgttaccg tcctcggcgt ccctaccgct 2820
cctagctccg ttaccttcaa cggcgccgct gttcctcgcg cttccgtcgc ttacaactct 2880
acctccaaga ccctgttcgt tggcggcctg caagacttca ccaagaccgg cgcttgggct 2940
gacaagtggg ttctcaagtg g 2961

<210> 6
<211> 987
<212> PRT

<213> Paecilomyces variotii

<400> 6

```
Met Gly Gly Phe Thr His Tyr Met Leu Ala Ser Ala Trp Leu Pro Leu
1               5                   10                  15

Thr Leu Gly Ala Ala Ile Ser Pro Ala Ala Thr Ser Ser Thr Ala Ser
            20                  25                  30

Trp Gly Pro Val Phe Thr Val Pro Ala Ser Ala Asp Glu Gly Ala Gln
        35                  40                  45

Leu Ile Ala Asn Ile Asn Asp Pro Gln Ser Val Asn Ala Gln Thr Val
    50                  55                  60

Cys Pro Gly Tyr Val Ala Ser Asn Val Gln Asn Asn Glu Phe Gly Phe
65                  70                  75                  80

Thr Ala Thr Leu Asn Leu Ala Gly Lys Ala Cys Asn Val Tyr Gly Thr
                85                  90                  95

Asp Val Asp Ser Leu Asn Leu Thr Val Gln Tyr Gln Ala Ser Asp Arg
            100                 105                 110

Leu Asn Ile Asn Ile Gly Pro Ala His Val Asp Ala Ser Asn Glu Ser
        115                 120                 125

Trp Tyr Ile Leu Ser Asp Asp Leu Val Tyr Lys Pro Thr Val Asp Gly
    130                 135                 140

Thr Ala Ser Ile Ser Gln Ser Asp Leu Leu Val Ser Trp Ser Asn Glu
145                 150                 155                 160

Pro Ser Phe Asn Phe Lys Val Ile Arg Lys Ala Asn Lys Asp Val Leu
                165                 170                 175

Phe Asn Thr Glu Gly Thr Val Leu Val Tyr Glu Asn Gln Phe Ile Glu
            180                 185                 190

Phe Val Ser Ala Leu Pro Glu Asn Tyr Asn Leu Tyr Gly Leu Gly Glu
        195                 200                 205

Arg Ile His Gly Leu Arg Leu Gly Asn Asn Phe Thr Ala Thr Thr Tyr
    210                 215                 220

Ala Ala Asp Ala Ala Asp Pro Ile Asp Ala Asn Ile Tyr Gly Thr His
225                 230                 235                 240
```

```
Pro Phe Tyr Leu Asp Thr Arg Tyr Tyr Glu Val Asp Ser Lys Gln Gly
                245                 250                 255

Thr Tyr Thr Leu Leu Thr Thr Asn Glu Thr Asp Gln Ser Lys Glu Tyr
            260                 265                 270

Thr Ser Phe Ser His Gly Val Phe Leu Arg Asn Ala His Gly Gln Glu
        275                 280                 285

Val Leu Leu Arg Pro Glu Gly Ile Thr Trp Arg Thr Leu Gly Gly Ser
    290                 295                 300

Ile Asp Leu Tyr Phe Tyr Ser Gly Pro Thr Gln Ala Asp Val Thr Arg
305                 310                 315                 320

Ser Tyr Gln Thr Ser Thr Val Gly Leu Pro Thr Met Gln Gln Tyr Tyr
                325                 330                 335

Thr Phe Gly Tyr His Gln Cys Arg Trp Gly Tyr Gln Asn Trp Ser Val
            340                 345                 350

Met Ala Asp Val Val Ser Ser Phe Ala Lys Phe Gln Ile Pro Leu Glu
        355                 360                 365

Thr Ile Trp Ser Asp Ile Asp Tyr Met Asn Ala Tyr Arg Asp Phe Glu
    370                 375                 380

Asn Asp Pro Ile Arg Phe Ser Tyr Ser Glu Gly Ala Glu Phe Leu Gly
385                 390                 395                 400

Gln Leu His Glu Asn Gly Gln His Trp Val Pro Ile Val Asp Ser Ala
                405                 410                 415

Ile Tyr Ile Pro Asn Ser Glu Asn Ala Ser Asp Ala Tyr Asp Val Tyr
            420                 425                 430

Thr Arg Gly Glu Ala Asp Gly Val Trp Met Thr Asn Pro Asp Gly Ser
        435                 440                 445

Leu Tyr Ile Gly Ala Val Trp Pro Gly Tyr Thr Val Phe Pro Asp Trp
    450                 455                 460

His Asn Pro Lys Ala His Glu Phe Trp Ser Asn Glu Ile Ala Thr Trp
465                 470                 475                 480

His Gln Lys Val Ala Phe Asp Gly Ile Trp Ile Asp Met Ser Glu Val
                485                 490                 495
```

```
Ser Ser Phe Cys Val Gly Ser Cys Gly Thr Gly Asn Leu Thr Leu Asn
            500                 505                 510

Pro Val His Pro Ser Phe Leu Leu Pro Gly Glu Pro Gly Ala Val Ile
        515                 520                 525

Tyr Asp Tyr Pro Glu Ser Phe Asn Val Thr Asn Ser Thr Glu Ala Ala
    530                 535                 540

Ser Ala Ser Ala Ala Ser Val Ser Gln Ala Ala Ala Thr Ala Ser Ala
545                 550                 555                 560

Ser Ala Ser Thr Thr Thr Ser Tyr Leu Arg Thr Thr Pro Thr Pro Gly
                565                 570                 575

Val Arg Asp Val Asn His Pro Pro Tyr Val Ile Asn Asn Val Gln Pro
            580                 585                 590

Gly His Asp Leu Ala Val His Ala Val Ser Pro Asn Ala Thr His Ile
        595                 600                 605

Asp Gly Val Ser Glu Tyr Asp Val His Asn Leu Trp Gly Tyr Gln Ile
    610                 615                 620

Leu Asn Ala Thr Tyr His Gly Leu Leu Lys Val Trp Glu Asp Lys Arg
625                 630                 635                 640

Pro Phe Ile Ile Gly Arg Ser Thr Phe Ala Gly Ser Gly Lys Trp Ala
                645                 650                 655

Gly His Trp Gly Gly Asp Asn Thr Ser Leu Trp Ala Tyr Met Phe Phe
            660                 665                 670

Ser Ile Pro Gln Ala Leu Ser Phe Ser Leu Phe Gly Ile Pro Met Phe
        675                 680                 685

Gly Val Asp Thr Cys Gly Phe Asn Gly Asn Ser Asp Glu Glu Leu Cys
    690                 695                 700

Asn Arg Trp Met Gln Leu Ser Ala Phe Phe Pro Phe Tyr Arg Asn His
705                 710                 715                 720

Asn Val Leu Ser Ala Ile Ser Gln Glu Pro Tyr Val Trp Ala Ser Val
                725                 730                 735

Ile Asp Ala Ser Lys Ala Ala Met Lys Ile Arg Tyr Ala Leu Leu Pro
            740                 745                 750
```

```
Tyr Ile Tyr Thr Leu Phe Tyr Leu Ala His Thr Thr Gly Ser Thr Val
        755                 760                 765

Met Arg Ala Val Ser Trp Glu Phe Pro Asn Asp Pro Ser Leu Ala Ala
    770                 775                 780

Ile Asp Thr Gln Phe Leu Leu Gly Pro Ser Leu Met Val Val Pro Val
785                 790                 795                 800

Leu Glu Pro Gln Val Asp Tyr Val Lys Gly Val Phe Pro Gly Val Gly
                805                 810                 815

Asn Gly Glu Val Trp Tyr Asp Trp Tyr Thr Gln Ser Val Phe Asp Ala
            820                 825                 830

Lys Pro Gly Val Asn Thr Thr Ile Ser Ala Pro Leu Gly His Ile Pro
        835                 840                 845

Val Phe Val Arg Gly Gly Ser Ile Leu Pro Met Gln Glu Pro Ala Leu
    850                 855                 860

Thr Thr Arg Asp Ala Arg Lys Thr Pro Trp Ala Leu Leu Thr Ala Leu
865                 870                 875                 880

Gly Gly Asn Gly Thr Ala Ser Gly Gln Leu Tyr Ile Asp Asp Gly Glu
                885                 890                 895

Ser Ile Thr Pro Asn Ala Thr Leu Asn Val Asp Phe Val Ala Ser Asn
            900                 905                 910

Ser Asn Leu Val Ala Ser Pro Arg Gly Ser Trp Val Glu Lys Asn Pro
        915                 920                 925

Leu Ala Asn Val Thr Val Leu Gly Val Pro Thr Ala Pro Ser Ser Val
    930                 935                 940

Thr Phe Asn Gly Ala Ala Val Pro Arg Ala Ser Val Ala Tyr Asn Ser
945                 950                 955                 960

Thr Ser Lys Thr Leu Phe Val Gly Gly Leu Gln Asp Phe Thr Lys Thr
                965                 970                 975

Gly Ala Trp Ala Asp Lys Trp Val Leu Lys Trp
            980                 985
```

<210> 7
<211> 2961

<212> DNA
<213> Artificial Sequence

<220>
<223> Synthetic construct

<400> 7

```
atgggcggct tcacccacta catgctcgct tccgcttggc tgcctctgac cctgggcgcc      60
gccatctctc ccgctgctac cagcagcacc gcttcgtggg gccccgtttt caccgtcccc     120
gcctccgccg acgagggcgc tcagctcatt gccaacatta acgaccccca gtctgttaac     180
gcccagaccg tttgccccgg ctacgttgct tctaacgtcc agaacaacga gttcggcttc     240
accgccaccc tgaacctcgc cggcaaggct tgcaacgttt acggcaccga cgtcgatagc     300
ctgaacctga ccgtccagta ccaggcgagt gaccgactga acattaacat tggccccgcc     360
cacgttgacg cctccaacga gtcttggtac attctgagcg acgacctggt ttacaagcct     420
accgtggacg gcaccgcttc tatctctcag tctgacctgc tggttagctg gtctaacgag     480
ccttctttca acttcaaggt tattcgtaag gctaacaagg acgtcctgtt caacaccgag     540
ggcaccgtcc tcgtttacga gaaccagttc atcgagttcg tcagcgctct ccctgagaac     600
tacaacctgt acggcctggg cgagcgcatt cacggcctgc gactcggcaa caacttcacc     660
gccaccacct acgccgccga cgccgccgac cccatcgacg ctaacatata tggcacccac     720
cctttctacc tggacacccg atactacgag gttgactcca agcagggcac ctacaccctg     780
ctgaccacca acgagaccga ccagtctaag gaatacacct ctttctctca cggcgttttc     840
ctccgaaacg ctcacggcca ggaagttctg ctgcgccctg agggcatcac ctggcgaacc     900
ctgggcggca gcattgacct gtacttctac tccggcccta cccaggctga cgtcacccga     960
tcttaccaga ccagcaccgt tggcctgcct accatgcagc agtactacac cttcggctac    1020
caccagtgcc gatggggcta ccagaactgg tctgtcatgg ctgacgttgt tagctctttc    1080
gccaagttcc agattcctct ggagaccatt tggagcgaca tcgactacat gaacgcttac    1140
cgagacttcg agaacgaccc tatccgattc tcttactctg agggcgctga gttcctcggc    1200
cagctgcacg agaacggcca gcactgggtt cctattgttg actccgccat ctacatccct    1260
aacagcgaga acgccagcga cgcttacgac gtttacaccc gaggcgaggc cgacggcgtt    1320
tggatgacca accccgacgg cagcctgtac attggcgccg tttggcccgg ctacaccgtt    1380
ttccccgact ggcacaaccc taaggctcac gagttctggt ctaacgagat tgctacctgg    1440
caccagaagg tcgctttcga cggcatttgg attgacatgt ctgaggtcag ctctttctgc    1500
gttggctctt gcggcaccgg caacctgacc ctcaaccccg tccacccttc tttcctgctc    1560
cccggcgagc ccggcgctgt tatatatgac taccctgagt ctttcaacgt caccaactct    1620
accgaggctg cttctgcttc agccgcatcg gtctcccagg ctgctgctac cgcttctgcc    1680
```

```
tctgcctcaa ccaccacctc ttacctgcga accaccccta cccccggcgt ccgagacgtt      1740
aaccaccctc cttacgttat taacaacgtc cagcctggcc acgacctcgc cgtccacgcc      1800
gttagcccca acgctaccca cattgacggc gttagcgagt acgacgtcca caacctgtgg      1860
ggctaccaga ttctgaacgc tacctaccac ggcctcctca aggtttggga ggacaagcgc      1920
cctttcatta ttggccgaag caccttcgct ggcagcggca agtgggctgg ccactggggc      1980
ggcgacaaca cctctctgtg ggcttacatg ttcttcagca ttcctcaggc tctgtctttc      2040
agcctgttcg gcatccccat gttcggcgtt gacacctgcg gcttcaacgg caactccgac      2100
gaagaactgt gcaaccgatg gatgcagctg tctgctttct tccctttcta ccgaaaccac      2160
aacgtcctca gcgctattag ccaagaacct tacgtttggg cctccgtcat tgacgcttct      2220
aaggccgcca tgaagattcg atacgctctg ctgccttaca tatacaccct gttctacctc      2280
gctcacacca ccggcagcac cgtcatgcga gctgtctctt gggagttccc taacgaccct      2340
agcctcgccg ccattgacac ccagttcctc ctgggcccta gcctcatggt tgtccccgtc      2400
ctggagcctc aggttgacta cgttaagggc gttttccccg gcgttggcaa cggcgaggtt      2460
tggtacgact ggtacaccca gtctgttttc gacgccaagc ccggcgttaa caccaccatc      2520
agcgctcctc tcggccacat ccccgttttc gtccgaggcg gcagcattct gcctatgcaa      2580
gagcccgctc tgaccacccg cgacgctcga aagacccctt gggctctcct gaccgctctg      2640
ggcggcaacg gcaccgcttc tggccagctg tacatcgacg acggcgagtc tattaccccct      2700
aacgccaccc tgaacgtgga cttcgttgct tccaactcta acctcgttgc gtcacctcgg      2760
ggctcttggg ttgagaagaa ccctctcgct aacgttaccg tcctcggcgt ccctaccgct      2820
cctagctccg ttaccttcaa cggcgccgct gttcctcgcg cttccgtcgc ttacaactct      2880
acctccaaga ccctgttcgt tggcggcctg caagacttca ccaagaccgg cgcttgggct      2940
gacaagtggg ttctcaagtg g                                                2961
```